# EUROPEAN PATENT APPLICATION

(11) **EP 4 763 989 A1**
(43) Date of publication of application: **24.06.2026**
(21) Application number: 24854190.6
(22) Date of filing: 09.08.2024
(51) Int. Cl.: C12N 15/29, A01H 1/00, A01H 6/82, C12Q 1/6895

(54) **TOMATO PLANT HAVING RESISTANCE TO ROOT-KNOT NEMATODE AND LEAF MOLD**

(30) Priority: 16.08.2023 JP 2023132701
(71) Applicant: Sakata Seed Corporation, Yokohama-shi Kanagawa 224-0041 (JP)
(72) Inventor: ITAI, Tsuneatsu, Yokohama-shi, Kanagawa 224-0041 (JP); OKADA, Yoshitake, Yokohama-shi, Kanagawa 224-0041 (JP); MORITAMA, Yosuke, Yokohama-shi, Kanagawa 224-0041 (JP); MIZOGUCHI, Sentaro, Yokohama-shi, Kanagawa 224-0041 (JP); TSURUMI, Yuta, Yokohama-shi, Kanagawa 224-0041 (JP); HORIKIRI, Ryosuke, Yokohama-shi, Kanagawa 224-0041 (JP)
(74) Representative: Bandpay & Greuter
(86) International application number: PCT/JP2024/028724
(87) International publication number: WO 2025/037600

(57) **Abstract**

The present invention is directed to provide a tomato plant having resistance to root-knot nematode and leaf mold. An aspect of the present invention relates to a tomato plant having resistance to root-knot nematode and leaf mold and comprising an Mi-1 gene that confers resistance to root-knot nematode and a Cf-5 gene that confers resistance to leaf mold. The Mi-1 gene and the Cf-5 gene are in the coupling phase. Another aspect of the present invention relates to a DNA fragment comprising the Mi-1 gene and the Cf-5 gene.

## Description

### FIELD

The present invention relates to a tomato (Solanum lycopersicum) plant that has multiple commercially important genes, or more specifically, the Mi-1 gene that confers root-knot nematode resistance and the Cf-5 gene that confers leaf mold resistance, in the coupling phase (cis-phase) for coinheritance of these genes as a single gene.

### BACKGROUND

Tomato (Solanum lycopersicum) is a perennial plant of the genus Solanum in the family Solanaceae. Tomatoes are native to South America and widely cultivated throughout the world. Tomatoes are thought to have been introduced to Japan by the Dutch during the Edo period. Tomatoes are mainly used for their fruits, which are rich in, for example, vitamin C, lycopene, β-carotene, potassium, and vitamin E and have extra high nutritional value, and are regarded as one of the most important vegetables.

Plant varieties commonly include open-pollinated varieties and first filial generation varieties (hereafter referred to as F1). F1 varieties are widely used in major crops. F1 varieties grow well because of hybrid vigor (heterosis). F1 varieties are thus greatly advantageous in, for example, faster growth and higher yields. F1 varieties are expected to have improved resistance to pests and diseases or adaptability to the environment, such as cold resistance or heat resistance. An F1 variety includes heterozygotes with an identical genotype, and thus, its phenotype is highly uniform. This increases the marketability of the produce. An F1 variety can also accumulate useful traits of dominant genes of its parents and thus can be bred rapidly. With the advantages described above, F1 varieties are now dominant cultivars in major crops. Until the 1920s, edible tomatoes were mainly open-pollinated varieties. However, the 1930s saw a rapid shift to F1 varieties, and edible tomatoes today are mostly F1 varieties.

However, tomatoes are susceptible to many diseases (Non-Patent Literature 1). To counter these diseases, growers chemically control pathogens by using, for example, pesticides. However, such chemical control affects the environment and increases labor and cost of cultivation. Resistant varieties are thus the most effective method for disease control.

Diseases caused by nematodes and leaf mold are among the most significant diseases for growers. Nematodes inhabit diverse areas of the world, with their distribution ranging from the Arctic to the Antarctic. In addition to the wide distribution, various species of nematodes have diverse host ranges including plants and animals. Tomatoes are no exception, and Meloidogyne arenaria, M. incognita, and M. javanica are known as the three most important species that infect tomatoes.

Plants infected by root-knot nematodes form 1 to 10 mm galls or knots on the roots. Such changes promote transportation of nutrients from the plants to the nematodes and, at the same time, reduce the supply of water and nutrients to the plants, hindering the growth of the plants. The infected plants may also develop symptoms including retarded growth, wilting, and yellowing (chlorosis). Nematodes cause root galls to form in the growth period. Wilting is thus more noticeable, and fruit formation may also be affected.

Chemical control is effective for root-knot nematodes, and a nematicide, or methyl bromide, is highly effective in controlling nematodes in the soil. However, the use of methyl bromide is banned worldwide due to concerns about adverse effects on the human body and depletion of the ozone layer. Under such circumstances, tomato varieties resistant to the root-knot nematode are widely developed as an effective crop management strategy (Non-Patent Document 2).

The Mi-1 gene (Mi1.2 gene) is a root-knot nematode resistance gene that is located on chromosome 6 and has been introduced into a cultivated tomato species from a tomato wild species Solanum peruvianum through interspecific crossing. The gene was isolated in 1998, and its amino acid sequence and mRNA sequence (AAC67238.1 and AF039682.1) are registered in the National Center for Biotechnology Information (NCBI, https://www.ncbi.nlm.nih.gov/). Reliable DNA markers including Mi23 are later developed near the Mi-1 gene, and root-knot nematode resistant varieties are still actively developed using Mi-1 (Non-Patent Literature 2, Non-Patent Literature 3).

Leaf mold is a disease caused by the filamentous fungus Passalora fulva, also known as Fulvia fulva or Cladosporium fulvum, and is a worldwide issue. The mold grows lesions, and when a lesion covers the majority of a leaf, the leaf falls off. This major disease seriously damages yields and quality of fruits. Leaf mold control also includes chemical pathogen control using pesticides and other chemicals. However, such chemical control affects the human body and the environment and increases labor and cost of cultivation. Resistant varieties are thus the most effective method for disease control (Non-Patent Literature 4).

In breeding a tomato variety resistant to leaf mold, the resistance genes Cf-2, Cf-4, Cf-5, Cf-9, or others are used. However, more diverse races are known worldwide with the emergence of leaf mold that can infect the tomato plants having these resistance genes. Particularly, a leaf mold race that can break the leaf mold resistance based on Cf-9 gene that has been actively used in recent leaf mold resistance breeding has emerged as a major issue (Non-Patent Literature 5).

The leaf mold resistance gene Cf-5 is derived from a local tomato variety Solanum lycopersicum var. cerasiforme. The gene was isolated in 1998, and its amino acid sequence and full-length coding sequence (AAC78591.1 and AF053993.1) are registered in NCBI. Subsequently, a DNA marker was developed based on the genomic DNA sequence of the gene. This enabled efficient tomato leaf mold resistance breeding using the Cf-5 gene (Non-Patent Literatures 6 and 7).

A tomato plant holding the Cf-5 gene in its genome is resistant to leaf mold races that break the leaf mold resistance based on the Cf-9 gene. Currently, the emergence of leaf mold races that break the leaf mold resistance based on the Cf-5 gene is limited, and the Cf-5 gene is still a useful leaf mold resistance gene for tomato variety breeding (Non-Patent Literatures 8, 9, and 10).

Tomatoes are susceptible to many diseases. Thus, many disease resistance genes are preferably given in breeding varieties. Disease resistance genes are located on various chromosomes. Among the chromosomes, chromosome 6 is known for carrying, in addition to the root-knot nematode resistance gene Mi-1, the leaf mold resistance genes Cf-2 and Cf-5, many genes such as the powdery mildew resistance genes Ol-1 and Ol-4 or the yellow leaf curl resistance gene Ty-1, and is the chromosome with significant use in breeding (Non-Patent Literature 11).

When multiple resistance genes are given to an F1 variety in a common manner, each of the resistance genes may be introduced into one of the corresponding parental lines, and crossing may be performed. Giving more genes to the F1 variety involves an increased number of genes to be accumulated in one parental line. However, the gene loci carrying the resistance genes Mi-1, Cf-2, Cf-5, Ol-1, Ol-4, and Ty-1 located on chromosome 6 are all closely linked genetically. Thus, accumulation of the multiple genes in a single fixed line is greatly challenging. For efficient breeding, it is desirable to have a fixed line carrying multiple resistance genes on chromosome 6 (Patent Literature 2).

Additionally, many of disease resistance genes described as dominant genes turn out to have incomplete dominance, with more heterozygotes developing symptoms than homozygotes in a highly disease-conductive environment (Non-Patent Literature 12). To breed a strongly resistant F1 variety, the resistance genes may be given to both parental lines to cause the F1 generation to be homozygous for the given disease resistance genes. Linking multiple disease resistance genes located in repulsion phase into the coupling phase is thus useful.

Under such circumstances, attempts have been made to accumulate the genes on chromosome 6 in a fixed line. For example, Patent Literature 1 describes construction of a line having the Mi-1 gene and the Ty-1 gene in the coupling phase. More specifically, eight individuals each having the Mi-1 gene and the Ty-1 gene in the coupling phase are successfully constructed from 504 individuals of an F2 segregating population obtained from a hybrid of an individual that is homozygous for the Mi-1 gene and has no Ty-1 gene and an individual that is homozygous for the Ty-1 gene and has no Mi-1 gene.

Similarly, Patent Literature 2 reports construction of a line having the Ol-4 gene and the Ty-1 gene in the coupling phase. More specifically, five individuals, each having the Ty-1 gene and the Ol-4 gene in the coupling phase are successfully constructed from 500 individuals of a segregating population obtained by crossing an individual having no Ty-1 and Ol-4 genes with a hybrid of an individual that is homozygous for the Ty-1 gene having no Ol-4 gene and an individual that is homozygous for the Ol-4 gene having no Ty-1 gene.

### CITATION LIST

### PATENT LITERATURE

Patent Literature 1: Japanese Patent No. 5312423
Patent Literature 2: US Patent No. 11535861

### NON-PATENT LITERATURE

Non-Patent Literature 1: Brad Gabor; Wayne Wiebe, Tomato Diseases A Practical Guide for Seedmen, Growers and Agricultural Advisors. Seminis Vegetable Seeds, inc., 1997.
Non-Patent Literature 2: Zuebeyir Devran et al., Development of Molecular Markers for the Mi-1 Gene in Tomato Using the KASP Genotyping Assay. Hortic. Environ. Biotechnol. 2016, 57(2), 156-160.
Non-Patent Literature 3: Milligan et al., The Root Knot Nematode Resistance Gene Mi from Tomato Is a Member of the Leucine Zipper, Nucleotide Binding, Leucine-Rich Repeat Family of Plant Genes. The Plant Cell, August 1998, 10, 1307-1319.
Non-Patent Literature 4: University of Illinois Report on Plant Disease, Leaf Mold of Greenhouse Tomatoes. Integrated Pest Management at the University of Illinois, August 1989.
Non-Patent Literature 5: Differential Sets - Passalora fulva. International Seed Federation, May 2022.
Non-Patent Literature 6: Mark S. Dixon et al., The Tomato Cf-5 Disease Resistance Gene and Six Homologs Show Pronounced Allelic Variation in Leucine-Rich Repeat Copy Number. The Plant Cell, November 1998, 10, 1915-1925.
Non-Patent Literature 7: Yu Shuancang et al., A CAPS Marker for Leaf Mould Resistance Gene Cf-5 in Tomato. Molecular Plant Breeding, 2005, 3(1), 57-60.
Non-Patent Literature 8: Junichiro Enya et al., The First Occurrence of Leaf Mold of Tomato Caused by Races 4.9 and 4.9.11 of Passalora fulva (syn. Fulvia fulva) in Japan. Journal of General Plant Pathology, February 2009, 75(1), 76-79.
Non-Patent Literature 9: Kubota et al., Tomato Fixed Lines for Identifying Races of the Leaf Mold Pathogen. Ann. Rept. Kansai Pl. Prot. 2019, 61, 55-60.
Non-Patent Literature 10: Yuichiro Iida et al., Ecology and Control of Fulvia fulva. Plant Protection, 2019, 73(11).
Non-Patent Literature 11: Seifi et al., Linked, if Not the Same, Mi-1 Homologues Confer Resistance to Tomato Powdery Mildew and Root-Knot Nematodes. MPMI, 2011, 24(4), 441-450.
Non-Patent Literature 12: Yamakawa Kunio, Vegetable/Resistant Variety and Use. 1978, 53-54.
Non-Patent Literature 13: Van Wordragen et al., Genetic and Molecular Organization of the Short Arm and Pericentromeric Region of Tomato Chromosome 6. Euphytica, 1994, 79, 169-174.
Non-Patent Literature 14: Takayuki Mizukubo; Kazuyoshi Futai, Nematological Experimentation. Kyoto University Press, 2014.
Non-Patent Literature 15: Ohata Kan-ichi et al., Fundamentals of Crop Pathogen Research Techniques. Japan Plant Protection Association, 1995.

### BRIEF SUMMARY

### TECHNICAL PROBLEM

Although there is some genetic map for the Mi-1 gene and the Cf-5 gene which has been created based on relative positional relationship between for example, the respective genes and their adjacent molecular markers (Non-Patent Literature 13), the inventors know no example of developing of an actual plant line having the two genes in the coupling phase. No such tomato plant has been commercially sold or cultivated. Suppressed homologous chromosome recombination has been indicated in a region near the Mi-1 gene, and similarly, difficulty of homologous chromosome recombination in this region between Solanum peruvianum and Solanum lycopersicum is reported (Patent Literature 1). Thus, it proves that linking the Mi-1 gene and the Cf-5 gene in the coupling phase is challenging. Therefore, to develop an F1 variety with root-knot nematode resistance and leaf mold resistance, it has been necessary giving the Mi-1 gene to one crossing parent plant and giving the Cf-5 gene to the other crossing parent plant to accumulate the two genes in an F1 variety.

One or more aspects of the present invention are directed to providing a tomato plant having the Mi-1 gene and the Cf-5 gene in the coupling phase, which has not been developed due to particularly challenging chromosome recombination for disease resistance genes on chromosome 6, and thus facilitating breeding of an F1 variety in which a group of disease resistance genes located on chromosome 6 are accumulated. With the tomato plant having the Mi-1 gene and the Cf-5 gene in the coupling phase, one or more aspects of the present invention are directed to conveniently provide a product comprising a plant body, a seed, a seedling, or a portion of the plant body of the tomato plant having root-knot nematode resistance and leaf mold resistance.

### SOLUTION TO PROBLEM

In response to the above issue, the inventors performed repeated crossing and self-pollination of tomato plants with the Mi-1 gene and tomato plants with the Cf-5 gene and selection with, for example, DNA markers or inoculation tests to successfully construct tomato plants comprising the two genes in the coupling phase.

One or more aspects of the present invention are described below.
[1] A tomato plant having resistance to root-knot nematode and leaf mold, the tomato plant comprising:
   an Mi-1 gene conferring resistance to root-knot nematode; and
   a Cf-5 gene conferring resistance to leaf mold,
   the Mi-1 gene and the Cf-5 gene being in the coupling phase.
[2] The tomato plant according to [1], wherein
   the tomato plant comprises a DNA fragment comprising
   a nucleotide sequence encoding a polypeptide of an amino acid sequence of SEQ ID NO: 1, and
   a nucleotide sequence encoding a polypeptide of an amino acid sequence of SEQ ID NO: 2.
[3] The plant according to [1] or [2], wherein
   the Mi-1 gene and the Cf-5 gene are located on chromosome 6.
[4] The tomato plant according to any one of [1] to [3], wherein
   the tomato plant comprises a nucleotide sequence encoding a polypeptide of an amino acid sequence of SEQ ID NO: 1 and a nucleotide sequence encoding a polypeptide of an amino acid sequence of SEQ ID NO: 2 on the same genomic DNA of chromosome 6.
[5] The plant according to any one of [1] to [4], wherein
   the tomato plant is homozygous or heterozygous for the Mi-1 gene, and
   the tomato plant is homozygous or heterozygous for the Cf-5 gene.
[6] The plant according to any one of [1] to [5], wherein
   the Mi-1 gene is identified with a genotyping method using a primer set comprising a primer comprising a nucleotide sequence of SEQ ID NO: 3 and a primer comprising a nucleotide sequence of SEQ ID NO: 4 and/or a genotyping method using a primer set comprising a primer comprising a nucleotide sequence of SEQ ID NO: 5, a primer comprising a nucleotide sequence of SEQ ID NO: 6, and a primer comprising a nucleotide sequence of SEQ ID NO: 7, and
   the Cf-5 gene is identified with a genotyping method using a primer set comprising a primer comprising a nucleotide sequence of SEQ ID NO: 8 and a primer comprising a nucleotide sequence of SEQ ID NO: 9.
[7] The plant according to any one of [1] to [6], wherein
   the tomato plant is resistant to at least one species of root-knot nematodes in a genus Meloidogyne and resistant to at least one race in a Passalora fulva race group that cannot infect tomato plants expressing the Cf-5 gene.
[8] The tomato plant according to any one of [1] to [7], wherein
   the tomato plant has root-knot nematode resistance and leaf mold resistance derived from a tomato plant identified by an accession number FERM BP-22476 or FERM BP-22477.
[9] The tomato plant according to any one of [1] to [8], wherein
   the tomato plant is a hybrid obtained from a tomato plant identified by an accession number FERM BP-22476 or FERM BP-22477 as a parental line, or progeny of the hybrid plant.
[10] The tomato plant according to any one of [1] to [9], wherein
   the tomato plant comprises genomic DNA of chromosome 6 comprising
   (1) a nucleotide sequence of a portion comprising the Mi-1 gene and the Cf-5 gene in the genomic DNA of chromosome 6 of a tomato plant identified by an accession number FERM BP-22476 or FERM BP-22477, or
   (2) a nucleotide sequence being 50% or greater identical to the nucleotide sequence defined in (1) and comprising a nucleotide sequence encoding a polypeptide of an amino acid sequence of SEQ ID NO: 1 and a nucleotide sequence encoding a polypeptide of an amino acid sequence of SEQ ID NO: 2.
[11] The tomato plant according to [10], wherein
   the portion comprising the Mi-1 gene and the Cf-5 gene in the genomic DNA of chromosome 6 of the tomato plant identified by the accession number FERM BP-22476 or FERM BP-22477 is included in a region from a base identified by ch06_2151188 to a base identified by ch06_2742590 in the genomic DNA of chromosome 6 of the tomato plant identified by the accession number FERM BP-22476 or FERM BP-22477.
[12] A portion of a plant body of the tomato plant according to any one of [1] to [11].
[13] The portion according to [12], wherein
   the portion is a tomato plant cell without regeneration potential, and the tomato plant cell comprises a DNA fragment comprising a nucleotide sequence encoding a polypeptide of an amino acid sequence of SEQ ID NO: 1 and a nucleotide sequence encoding a polypeptide of an amino acid sequence of SEQ ID NO: 2.
[14] A fruit of the tomato plant according to any one of [1] to [11].
[15] A seed of the tomato plant according to any one of [1] to [11].
[16] A DNA fragment, comprising:
   an Mi-1 gene; and
   a Cf-5 gene.
[17] The DNA fragment according to [16], wherein
   the DNA fragment comprises a nucleotide sequence encoding a polypeptide of an amino acid sequence of SEQ ID NO: 1 and a nucleotide sequence encoding a polypeptide of an amino acid sequence of SEQ ID NO: 2.
[18] The DNA fragment according to [16] or [17], wherein
   the DNA fragment comprises
   (1) a nucleotide sequence of a portion comprising the Mi-1 gene and the Cf-5 gene in the genomic DNA of chromosome 6 of the tomato plant identified by the accession number FERM BP-22476 or FERM BP-22477, or
   (2) a nucleotide sequence being 50% or greater identical to the nucleotide sequence defined in (1) and comprising a nucleotide sequence encoding a polypeptide of an amino acid sequence of SEQ ID NO: 1 and a nucleotide sequence encoding a polypeptide of an amino acid sequence of SEQ ID NO: 2.
[19] The DNA fragment according to [18], wherein
   the portion comprising the Mi-1 gene and the Cf-5 gene in the genomic DNA of chromosome 6 of the tomato plant identified by the accession number FERM BP-22476 or FERM BP-22477 is included in a region from a base identified by ch06_2151188 to a base identified by ch06_2742590 in the genomic DNA of chromosome 6 of the tomato plant identified by the accession number FERM BP-22476 or FERM BP-22477.
[20] A method for producing F1 seeds from the tomato plant according to any one of [1] to [11], the method comprising:
   crossing the tomato plant according to any one of [1] to [11] with another of the tomato plant according to any one of [1] to [11] or with a plant capable of being crossed with the tomato plant according to any one of [1] to [11]; and
   harvesting F1 seeds from an individual obtained by the crossing.
[21] A method for producing a tomato plant having resistance to root-knot nematode and leaf mold and comprising an Mi-1 gene and a Cf-5 gene in the coupling phase, the method comprising:
   self-pollinating the tomato plant according to any one of [1] to [11]; and
   further repeatedly self-pollinating an individual obtained by the self-pollinating for one or more generations.
[22] A method for producing a tomato plant having resistance to root-knot nematode and leaf mold and comprising an Mi-1 gene and a Cf-5 gene in the coupling phase, the method comprising:
   crossing the tomato plant according to any one of [1] to [11] with a first tomato plant;
   obtaining a segregating population by self-pollinating or back-crossing an F1 individual obtained by the crossing or by interspecifically or intraspecifically crossing the F1 individual with a second tomato plant different from the first tomato plant used in the crossing; and
   selecting a tomato plant having the Mi-1 gene and the Cf-5 gene in the coupling phase from the segregating population.
[23] The method according to [22], wherein
   the selecting comprises at least one of
   detecting the Mi-1 gene with a genotyping method using a primer set comprising a primer comprising a nucleotide sequence of SEQ ID NO: 3 and a primer comprising a nucleotide sequence of SEQ ID NO: 4,
   detecting the Mi-1 gene with a genotyping method using a primer set comprising a primer comprising a nucleotide sequence of SEQ ID NO: 5, a primer comprising a nucleotide sequence of SEQ ID NO: 6, and a primer comprising a nucleotide sequence of SEQ ID NO: 7,
   detecting the Cf-5 gene with a genotyping method using a primer set comprising a primer comprising a nucleotide sequence of SEQ ID NO: 8 and a primer comprising a nucleotide sequence of SEQ ID NO: 9,
   confirming resistance to root-knot nematode, or
   confirming resistance to leaf mold.
[24] A method for manufacturing a product comprising a plant body, a seed, a seedling, or a portion of the plant body of a tomato plant, the method comprising:
   confirming that the tomato plant according to any one of [1] to [11] has one trait selected from the group consisting of root-knot nematode resistance, leaf mold resistance, presence of an Mi-1 gene, and presence of a Cf-5 gene; and
   preparing a product comprising a plant body, a seed, a seedling, or a portion of the plant body of the tomato plant confirmed to have the one trait.
[25] The method according to [24], wherein
   the product comprises a seed of the tomato plant and a wrapping material containing the seed, or the product comprises a seedling of the tomato plant and a tray containing the seedling.
[26] A product comprising a plant body, a seed, a seedling, or a portion of the plant body of a tomato plant manufactured with the method according to [24] or [25].
[27] The tomato plant according to any one of [1] to [11] obtained with a method not solely being an essentially biological method.
[28] The method according to [21], further comprising:
   selecting a tomato plant having the Mi-1 gene and the Cf-5 gene in the coupling phase from an individual obtained by the self-pollinating and/or an individual obtained by the further self-pollinating performed repeatedly for one or more generations.
[29] The method according to [28], wherein
   the selecting comprises at least one of
   detecting the Mi-1 gene with a genotyping method using a primer set comprising a primer comprising a nucleotide sequence of SEQ ID NO: 3 and a primer comprising a nucleotide sequence of SEQ ID NO: 4,
   detecting the Mi-1 gene with a genotyping method using a primer set comprising a primer comprising a nucleotide sequence of SEQ ID NO: 5, a primer comprising a nucleotide sequence of SEQ ID NO: 6, and a primer comprising a nucleotide sequence of SEQ ID NO: 7,
   detecting the Cf-5 gene with a genotyping method using a primer set comprising a primer comprising a nucleotide sequence of SEQ ID NO: 8 and a primer comprising a nucleotide sequence of SEQ ID NO: 9,
   confirming resistance to root-knot nematode, or
   confirming resistance to leaf mold.
[30] A method for selecting a tomato plant having resistance to root-knot nematode and leaf mold and comprising an Mi-1 gene and a Cf-5 gene in the coupling phase, the method comprising:
   (1) analyzing, with one or more assays, a tested tomato plant for presence of the Mi-1 gene and the Cf-5 gene on genomic DNA of one chromosome in the tested tomato plant; and
   (2) selecting a tested tomato plant confirmed to possess the Mi-1 gene and the Cf-5 gene present on the genomic DNA of the chromosome.
[31] The method according to [30], wherein
   the one or more assays in (1) include
   determining, based on a genotyping method for detecting a DNA marker of the Mi-1 gene, whether the Mi-1 gene is present and, when the Mi-1 gene is present, whether the tested tomato plant is heterozygous or homozygous for the Mi-1 gene, and
   determining, based on a genotyping method for detecting a DNA marker of the Cf-5 gene, whether the Cf-5 gene is present, and when the Cf-5 gene is present, the tested tomato plant is heterozygous or homozygous for the Cf-5 gene.
[32] The method according to [31], wherein
   the DNA marker of the Mi-1 gene and the DNA marker of the Cf-5 gene are each independently at least one selected from the group consisting of an SNP marker, an SSR marker, an RFLP marker, a SCAR marker, a CAPS marker, and a KASP^{™} marker.
[33] The method according to [31], wherein
   the DNA marker of the Mi-1 gene is at least one selected from the group consisting of an Mi23 marker and an Mi-1K marker, and the DNA marker of the Cf-5 gene is a Cf-5 marker.
[34] The method according to [33], wherein
   the Mi23 marker comprises a sequence distinguishable with a genotyping method using a primer set of SEQ ID NO: 3 and SEQ ID NO: 4,
   the Mi-1K marker comprises a sequence distinguishable with a genotyping method using a primer set of SEQ ID NO: 5, SEQ ID NO: 6, and SEQ ID NO: 7, and
   the Cf-5 marker comprises a sequence distinguishable with a genotyping method using a primer set of SEQ ID NO: 8 and SEQ ID NO: 9.
[35] The method according to any one of [30] to [34], wherein
   the one or more assays in (1) include
   determining, based on a genotyping method using a primer set of SEQ ID NO: 3 and SEQ ID NO: 4 and/or a primer set of SEQ ID NO: 5, SEQ ID NO: 6, and SEQ ID NO: 7, whether the Mi-1 gene is present and, when the Mi-1 gene is present, whether the tested tomato plant is heterozygous or homozygous for the Mi-1 gene, and
   determining, based on a genotyping method using a primer set of SEQ ID NO: 8 and SEQ ID NO: 9, whether the Cf-5 gene is present, and when the Cf-5 gene is present, the tested tomato plant is heterozygous or homozygous for the Cf-5 gene.
[36] The method according to [31] or [35], wherein
   the one or more assays identify a tested tomato plant homozygous for the Mi-1 gene and heterozygous for the Cf-5 gene, a tested tomato plant heterozygous for the Mi-1 gene and homozygous for the Cf-5 gene, or a tested tomato plant homozygous for the Mi-1 gene and homozygous for the Cf-5 gene as the tomato plant with the Mi-1 gene and the Cf-5 gene present on genomic DNA of one chromosome.
[37] The method according to [30], wherein
   the one or more assays in (1) include reading genomic DNA of one homologous chromosome of chromosome 6 in a tested tomato plant and confirming the presence of a nucleotide sequence encoding a polypeptide of an amino acid sequence of SEQ ID NO: 1 and the presence of a nucleotide sequence encoding a polypeptide of an amino acid sequence of SEQ ID NO: 2.
[38] The method according to any one of [30] to [37], wherein
   the tested tomato plant is progeny of the tomato plant having resistance to root-knot nematode and leaf mold and comprising the Mi-1 gene and the Cf-5 gene in the coupling phase.

The present application claims priority to Japanese Patent Application No. 2023-132701, the contents of which are incorporated herein.

All publications, patents, and patent applications cited herein are incorporated by reference in their entirety.

### ADVANTAGEOUS EFFECTS

One or more aspects of the present invention provides a tomato plant comprising the Mi-1 gene and the Cf-5 gene in the coupling phase. The tomato plant according to one or more aspects of the present invention can be used as a parental line to cause F1 varieties to easily accumulate multiple resistance genes located on chromosome 6. This allows development of products with high industrial value.

### BRIEF DESCRIPTION OF DRAWINGS

FIG. 1 is a schematic diagram of chromosome 6 of a tomato plant, illustrating the positional relationship between the Mi-1 gene, the Cf-5 gene, and the respective DNA markers (Example 2).
FIG. 2 is a photograph of tomato plant seedlings of a deposited line (accession number FERM BP-22476) on day 18 after inoculation of leaf mold race 2.4.9 in Example 5.
FIG. 3 is a photograph of tomato plant seedlings of a tomato line SKTom-C that is homozygous for the Mi-1 genes and has no Cf-5 gene, showing the seedlings on day 18 after inoculation of leaf mold race 2.4.9 in Example 5.
FIG. 4 is a photograph of leaves of tomato plant seedlings, showing the leaves of a deposited line (accession number FERM BP-22476) on day 18 after inoculation of leaf mold race 2.4.9 in Example 5 in the upper part, and the leaves of the tomato line SKTom-C that is homozygous for the Mi-1 gene and has no Cf-5 gene on day 18 after inoculation of leaf mold race 2.4.9 in Example 5 in the lower part.

### DETAILED DESCRIPTION

### Seed Deposition

In relation to the present disclosure, seeds of two tomato plant lines were deposited to National Institute of Technology and Evaluation Patent Microorganisms Depositary (#120, 2-5-8 Kazusakamatari, Kisarazu-shi, Chiba 2920818) and have the accession numbers as seen below.

### (Deposited line 1)

Accession number: FERM BP-22476
Identification label: SSC-TOM-23-001
Deposition date: May 12, 2023
(Deposited line 2)
Accession number: FERM BP-22477
Identification label: SSC-TOM-23-002
Deposition date: May 12, 2023

In one or more embodiments of the present disclosure, tomato plants are plants classified in the tomato clade in the genus Solanum (Bedinger et al., 2011). Cultivated tomato species include Solanum lycopersicum, and wild species tomatoes include Solanum peruvianum. Local variety tomatoes include Solanum lycopersicum var. cerasiforme. Cultivated species herein are plant lines for cultivation, including F1 varieties as well as open-pollinated varieties.

Root-knot nematodes herein are nematodes in the genus Meloidogyne.

Leaf mold herein is a disease caused by the filamentous fungus, Passalora fulva, for which multiple races are known.

ChX_Y (X and Y are integrates) or a base identified by chX_Y of tomato plants herein refers to a base corresponding to the Y-th base on a chromosome numbered X (also referred to as chromosome X) in a reference genome (SL4.0) available at Sol Genomics Network (https://solgenomics.net/) among base sequences of genomic DNA of the tomato plant. In a specific tomato plant, a base corresponding to the Y-th base on chromosome X in the reference genome available at Sol Genomics Network can be determined by aligning a base sequence of the genomic DNA of the specific tomato plant with base sequences of the reference genome from Sol Genomics Network to achieve the highest homology (sequential identity). Chromosome X of the specific tomato plant can thus be indicated as chromosome X of the tomato plant determined by aligning the base sequence of the genomic DNA of the specific tomato plant with base sequences of the reference genome from Sol Genomics Network to achieve the highest homology (sequential identity).

A chromosome herein includes a chromosome entirely or partially. In other words, a part of a chromosome may also be simply referred to as a chromosome.

An X gene locus is a position on a chromosome occupied with the X gene. A tomato plant having an X gene locus herein is thus a tomato plant having a chromosome including a position occupied with the X gene and is interchangeable with a tomato plant having the X gene.

Being near herein is easily understood as a specific distant by those skilled in the art based on the relationship between the marker position and the root-knot nematode resistance gene Mi-1 or between the marker position and the leaf mold resistance gene Cf-5, the relationship between the root-knot nematode resistance gene Mi-1 and the leaf mold resistance gene Cf-5, and common knowledge among those skilled in the art. An example distance may be, although susceptible to analysis conditions, a distance of about 10 cM or less (e.g., 7 cM).

Thus, an Mi23 marker identified by a primer set of SEQ ID NOs: 3 and 4 and/or an Mi-1K marker identified by a primer set of SEQ ID NOs: 5, 6, and 7 shown in Table 1 in the examples can be used to estimate or confirm the presence of the Mi-1 gene located near the gene loci indicated by the markers.

The coupling phase and the cis-phase herein refer to a genetic state in which alleles at two different loci are linked to each other on a single (homologous) chromosome. For example, when both the Mi-1 gene and the Cf-5 gene are located on one chromosome of homologous chromosomes, the alleles are in the coupling phase or cis-phase. In contrast, when the Mi-1 gene and the Cf-5 gene are located on different homologous chromosomes of a single homologous pair, the genes are in repulsion phase or trans-phase. The coupling phase or cis-phase also includes a state in which the genes or base sequences originally located on the same gene locus in repulsion phase or trans-phase are linked on a single DNA fragment, as well as on a single chromosome or chromosome fragment.

In the present disclosure, the "production method" can also be rephrased as a "generation method", a "developing method", a "growth method", or a "breeding method". Therefore, the terms "production", "generation", "development", "growth", and "breeding" used herein are interchangeably used.

In the present disclosure, a "part of a plant body" includes cells, tissues or organs of the plant body. Examples thereof include fruits, seeds, flowers, pollen, anthers, leaves, stems, roots, embryos, hypocotyls, meristem cells, and calluses. Other than the above, protoplasts obtained from cells of the plant body are also included. Tomato plants have been reported to undergo regeneration from the hypocotyls, explants from young unfolding leaves, seeds, shoot apical meristems, and primordia of stems and leaves among the plant cells, tissues, or organs described above (Kita, 1987; Toyoda, 1987; and Sakata, 1991). Particularly, a tomato fruit consists of an epicarp (including epidermal cells), a mesocarp, an endocarp, seeds, a placenta, locules, a vascular bundle, a pedicle, sepals, and other parts (Matsui, 1981). However, the inventors know no report that the fruit, except seeds, re-generates with totipotency from sufficiently differentiated plant cells, tissues, and organs.

### <Tomato Plant Resistant to Root-knot Nematode and Leaf Mold>

A tomato plant resistant to root-knot nematode and leaf mold according to one or more embodiments of the present disclosure possesses the root-knot nematode resistance gene Mi-1 and the leaf mold resistance gene Cf-5 linked in the coupling phase. The tomato plant resistant to root-knot nematode and leaf mold according to one or more embodiments of the present disclosure includes the Mi-1 gene and the Cf-5 gene in the coupling phase in a manner in which the Mi-1 gene and the Cf-5 gene can be expressed.

The Mi-1 gene confers resistance to a wide range of root-knot nematode species including Meloidogyne arenaria (arenaria root-knot nematode), Meloidogyne incognita (Southern root-knot nematode), and Meloidogyne javanica (Javanese root-knot nematode). Although the Mi-1 gene is known to lose function in an environment in which the soil temperature is 28 °C or higher, the Mi-1 gene is still widely used in breeding a variety and maintains a key role for its effectiveness to a wide range of root-knot nematode species.

The Mi-1 gene is registered in NCBI with its amino acid sequence (SEQ ID NO: 1) as AAC67238.1 and its mRNA nucleotide sequence as AF039682.1 (SEQ ID NO: 10). It is well known in the art that nematode resistance conferred by Mi-1 is attributable to this amino acid sequence. In the mRNA sequence of the Mi-1 gene shown as SEQ ID NO: 10 and a DNA sequence in which all Us in SEQ ID NO: 10 are replaced by Ts, a partial nucleotide sequence from the 87th base to the 3860th base is a coding sequence of the amino acid sequence of SEQ ID NO: 1.

The Mi-1 gene is dominantly inherited. The tomato plant according to one or more embodiments of the present disclosure may thus be a tomato plant homozygous for the Mi-1, or in other words, having the Mi-1 gene homozygously, or may be a tomato plant heterozygous for the Mi-1 gene.

The Cf-5 gene confers resistance to Passalora fulva races 0 (Pf:0), 2 (Pf:A), 4 (Pf:B), 2.4 (Pf:C), 2.9 (Pf:F), 9 (Pf:G), 4.9 (Pf:H), and 2.6.9 (Pf:J) defined by the International Seed Federation (ISF) or race 2.4.9 and other races reportedly occurring in Japan.

The Cf-5 gene is registered in NCBI with its amino acid sequence (SEQ ID NO: 2) as AAC78591.1 and its nucleotide sequence for the full-length coding sequence as AF053993.1 (SEQ ID NO: 11). It is well known in the art that leaf mold disease resistance conferred by Cf-5 is attributable to this amino acid sequence. In the nucleotide sequence of the Cf-5 gene shown as SEQ ID NO: 11, a partial nucleotide sequence from the 654th base to the 3560th base is a coding sequence of the amino acid sequence of SEQ ID NO: 2.

The Cf-5 gene is a dominantly inherited gene. The tomato plant according to one or more embodiments of the present disclosure may thus be a tomato plant homozygous for the Cf-5 gene, or in other words, having the Cf-5 gene homozygously, or may be a tomato plant heterozygous for the Cf-5 gene.

The tomato plant resistant to root-knot nematode and leaf mold according to one or more embodiments of the present disclosure can thus be produced by introducing DNA fragments comprising the Mi-1 gene and the Cf-5 gene linked in the coupling phase into the genomic DNA of a tomato plant. The DNA fragments comprising the Mi-1 gene and the Cf-5 gene linked in the coupling phase may be introduced to any part of a cell and may be introduced into or outside the chromosomes. For example, the tomato plant resistant to root-knot nematode and leaf mold according to one or more embodiments of the present disclosure includes a tomato plant in which a region on chromosome 6 corresponding to the DNA fragment comprising the Mi-1 gene and the Cf-5 gene linked in the coupling phase is replaced by the fragment, or a tomato plant in which, due to translocation or other phenomena, the DNA fragment comprising the Mi-1 gene and the Cf-5 gene linked in the coupling phase is introduced into a region other than the region corresponding to the Mi-1 and Cf-5 gene loci in chromosome 6 of the tomato plant.

The DNA fragment comprising the Mi-1 gene and the Cf-5 gene linked in the coupling phase may be, for example, a fragment that exhibits the Mi-1 genotype based on a genotyping method using a primer set of SEQ ID NOs: 3 and 4 and/or a primer set of SEQ ID NOs: 5, 6, and 7 and the Cf-5 genotype based on a genotyping method using a primer set of SEQ ID NOs: 8 and 9. However, the DNA fragment is not limited to the above fragment, and may be any single DNA fragment comprising a nucleotide sequence encoding a polypeptide of an amino acid sequence of SEQ ID NO: 1 and a nucleotide sequence encoding a polypeptide of an amino acid sequence of SEQ ID NO: 2 in a manner in which the nucleotide sequences can be expressed. In other words, the tomato plant resistant to root-knot nematode and leaf mold according to one or more embodiments of the present disclosure is preferably a tomato plant comprising, in its genomic DNA, a DNA fragment comprising the nucleotide sequences encoding the polypeptides of the amino acid sequences of SEQ ID NOs: 1 and 2 in a manner in which the nucleotide sequences can be expressed. The DNA fragment comprising the Mi-1 gene and the Cf-5 gene linked in the coupling phase may also be a DNA fragment that exhibits the Mi-1 genotype based on the genotyping method using the primer set of SEQ ID NOs: 3 and 4 and/or the primer set of SEQ ID NOs: 5, 6, and 7 and the Cf-5 genotype based on the genotyping method using the primer set of SEQ ID NOs: 8 and 9.

In one or more embodiments of the present disclosure, the Mi-1 gene is thus identified based on a genotyping method using a primer set comprising a primer comprising the nucleotide sequence of SEQ ID NO: 3 and a primer comprising the nucleotide sequence of SEQ ID NO: 4 and/or a genotyping method using a primer set comprising a primer comprising the nucleotide sequence of SEQ ID NO: 5, a primer comprising the nucleotide sequence of SEQ ID NO: 6, and a primer comprising the nucleotide sequence of SEQ ID NO: 7. In one or more embodiments of the present disclosure, the Cf-5 gene is identified based on a genotyping method using a primer set comprising a primer comprising the nucleotide sequence of SEQ ID NO: 8 and a primer comprising the nucleotide sequence of SEQ ID NO: 9. The markers used in these genotyping methods are codominant markers and it is possible to determine whether a tomato plant is homozygous or heterozygous for each of the Mi-1 gene and the Cf-5 gene.

The genotyping methods will now be described. A primer comprising a nucleotide sequence of SEQ ID NO: N herein is a primer comprising the nucleotide sequence of SEQ ID NO: N at the 3'-end. The primer may further comprise another nucleotide sequence added to the 5'-end of the nucleotide sequence of SEQ ID NO: N. Each primer can include a labeling substance as appropriate.

The primer set comprising the primer comprising the nucleotide sequence of SEQ ID NO: 3 and the primer comprising the nucleotide sequence of SEQ ID NO: 4 can amplify, through a nucleic acid amplification reaction using DNA extracted from a tomato plant as a template, the nucleotide sequence of Mi23 that is specifically linked to the Mi-1 gene and the nucleotide sequence of another allele of Mi23 linked to another allele of the Mi-1 gene as amplified products of different sizes that are distinguishably detectable. The genotyping method using the primer set can thus determine whether the Mi-1 gene is present and, when the Mi-1 gene is present, whether the tomato plant is heterozygous or homozygous for the Mi-1 gene.

The primer set comprising the primer comprising the nucleotide sequence of SEQ ID NO: 5 (Mi1K_1F), the primer comprising the nucleotide sequence of SEQ ID NO: 6 (Mi1K_2F), and the primer comprising the nucleotide sequence of SEQ ID NO: 7(Mi1K_R) can amplify, through the nucleic acid amplification reaction using DNA extracted from a tomato plant, the nucleotide sequence of Mi-1K that is specifically linked to the Mi-1 gene as amplified products of Mi1K_1F and Mi1K_R and the nucleotide sequence of another allele of Mi-1K linked to another allele of the Mi-1 gene as amplified products of Mi1K_2F and Mi1K_R. Mi1K_1F comprises a nucleotide sequence (tail sequence) added to 5'-end of the nucleotide sequence of SEQ ID NO: 5, and Mi1K_2F comprises a nucleotide sequence (tail sequence) added to 5'-end of the nucleotide sequence of SEQ ID NO: 6. The tail sequences are distinguishably detectable. In the nucleic acid amplification reaction, the amplified product of Mi1K_1F and Mi1K_R and the amplified product of Mi1K_2F and Mi1K_R can be distinguished from each other by using probes. Each probe is a nucleotide sequence that is annealed to the corresponding tail sequence with a labeling substance added to distinguish the tail sequences. The genotyping method using the primer set can thus determine whether the Mi-1 gene is present and, when the Mi-1 gene is present, whether the tomato plant is heterozygous or homozygous for the Mi-1 gene.

The primer set comprising the primer comprising the nucleotide sequence of SEQ ID NO: 8 and the primer comprising the nucleotide sequence of SEQ ID NO: 9 can amplify, through the nucleic acid amplification reaction using DNA extracted from a tomato plant as a template, a partial nucleotide sequence that is specifically included in the Cf-5 gene and the partial nucleotide sequence included in another allele of the Cf-5 gene. The resultant amplified products are digested differently by the restriction enzyme TaqI (in size and/or number of fragments after the TaqI treatment) and are distinguishably detectable. The genotyping method using the primer set and TaqI, as appropriate, can thus determine whether the Cf-5 gene is present and, when the Cf-5 gene is present, whether the tomato plant is heterozygous or homozygous for the Cf-5 gene.

The Mi-1 gene and the Cf-5 gene can be linked on a single DNA fragment with, in addition to a method using homologous chromosome recombination, methods well known in the art, such as a method of linking DNA fragments comprising the respective gene sequences in vitro with a restriction enzyme and a DNA ligase. Other methods include artificial gene synthesis services provided by, for example, Eurofin genomics. Such services allow artificial synthesis of a DNA sequence of the two genes linked to each other.

The DNA fragments comprising the respective gene sequences may be, in addition to fragments comprising DNA sequences derived from the genomic DNA, any fragments that allow stable gene expression, and may be fragments comprising DNA sequences of cDNA.

DNA fragments having the Mi-1 gene and the Cf-5 gene linked in the coupling phase may be fragments comprising the nucleotide sequences of the linked genes and may be of any length. The DNA fragments may be, for example, 700 kb or greater, or less than 700kb. The DNA fragments may be less than 650 kb, less than 400 kb, less than 200 kb, less than 100 kb, less than 50 kb, or less than 10kb.

The DNA fragments comprising the Mi-1 gene and the Cf-5 gene artificially linked in the coupling phase as described above can be amplified with a method well known in the art, such as cloning using, for example, vectors and Escherichia coli, or LA PCR^{™}. The vectors are, for example, BIBAC vectors (U.S. Patent No. 5733744) or large-capacity binary shuttle vectors (Japanese Patent No. 3350753). Kits comprising polymerase chain reaction (PCR) enzymes for LA PCR are sold by companies that supply products for molecular biology, such as Takara Bio. These kits allow amplification of long-chain DNA fragments of tens to hundreds kilobases.

When the DNA fragments comprising the expressible Mi-1 gene and the Cf-5 gene artificially linked in the coupling phase are used with, for example, the BIBAC vectors and the large-capacity binary shuttle vectors (Japanese Patent No. 3350753) in combination with a method well known in the art, such as a method using Agrobacterium, the Mi-1 gene and the Cf-5 gene artificially linked in the coupling phase are introduced into cells of a tomato plant. This can develop tomato plants having root-knot nematode resistance and leaf mold resistance.

As described above, the methods for linking and introducing include, in addition to methods using chromosome recombination in cross breeding, a technique of linking and introducing the two genes in vitro using DNA recombination techniques (gene recombination) or a technique of genetic engineering (GE), for example. However, crops developed through, for example, gene recombination or genetic engineering have not been widely accepted by the public.

An example of tomato plant resistant to root-knot nematode and leaf mold according to one or more embodiments of the present disclosure is a tomato plant that is developed using tomato plants having root-knot nematode resistance derived from the Mi-1 gene and tomato plants having leaf mold resistance derived from the Cf-5 gene as parental lines and is resistant to root-knot nematode and leaf mold with the Mi-1 gene and the Cf-5 gene linked in the coupling phase. Tomato plants include plants resulting from interspecific or intraspecific crosses within the genus Solanum (tomato clade), somatic cell hybrid plants generated by interspecific or intraspecific cell fusion within the genus Solanum (tomato clade), and graft hybrids obtained by interspecific or intraspecific grafting within the genus Solanum (tomato clade).

In a method for producing a tomato according to one or more embodiments of the present disclosure, cross breeding with chromosome recombination, for example, may use any tomato plants as parental lines when one of the parents is a tomato plant having root-knot nematode resistance derived from the Mi-1 gene and the other of the parent is a tomato plant having leaf mold resistance derived from the Cf-5 gene. The tomato plants are preferably varieties cultivated as crops. Tomato plants currently available in the Japanese domestic market are mainly, for example, large-fruit tomato varieties, medium-fruit tomato varieties, cherry tomato varieties, and rootstock tomato varieties. Any of these tomatoes that satisfy the conditions described above may be used. For example, F1 varieties, such as Reiyou and Gohoubi, sold in Japan by Sakata Seed Corporation are tomato plants that have one parent homozygous for the Mi-1 gene and the other parent homozygous for the Cf-5 gene, and thus are heterozygotes having the Mi-1 gene and the Cf-5 gene in repulsion phase (trans-phase). These tomato varieties can be used as parental lines for producing the tomatoes according to one or more embodiments of the present disclosure.

The tomato plants used as parental lines may be, as described above, any combination that includes a tomato plant having root-knot nematode resistance derived from the Mi-1 gene as one parent and a tomato plant having leaf mold resistance derived from the Cf-5 gene as the other parent. Such tomato plants used as parental lines can be selected from various types of commonly available tomato plants after confirming the presence of the root-knot nematode resistance derived from the Mi-1 gene or the leaf mold resistance derived from the Cf-5 gene with, for example, the genotyping methods or inoculation tests described herein or by reading base sequences. The tomato plants used as parental lines may not be the varieties owned by Sakata Seed Corporation.

"Progeny of a tomato plant" herein includes, in addition to descendants obtained by intraspecific crossing of the tomato plant, individuals obtained from the tomato plant as a parental line and their descendants, somatic cell hybrid plants obtained by cell fusion of the tomato plant's cells and another plant's cells and their descendants, and individuals obtained by grafting with the tomato plant as a rootstock or a scion and their descendants. "Descendants" include both the individuals obtained as intraspecific hybrids and the individuals obtained as interspecific hybrids. "Individuals obtained from (a plant as) a parental line" are individuals obtained by intraspecific or interspecific crossing with the plants as a parental line, cell fusion, tissue culture, or grafting.

The tomato plant resistant to root-knot nematode and leaf mold according to one or more embodiments of the present disclosure is any plant having the Mi-1 gene and the Cf-5 gene linked in the coupling phase and is preferably a tomato plant with traits of cultivated tomato species.

The tomato plant resistant to root-knot nematode and leaf mold according to one or more embodiments of the present disclosure is developed from, for example, the progeny of F1 individuals obtained by crossing of a tomato plant having the Mi-1 gene and no Cf-5 gene and a tomato plant having the Cf-5 gene and no Mi-1 gene. For example, a tomato plant that is homozygous for the Mi-1 gene and has no Cf-5 gene and a tomato plant that is homozygous for the Cf-5 gene and has no Mi-1 gene are crossed to produce seeds of tomato plants that are heterozygous for each of the Mi-1 gene and the Cf-5 gene in repulsion phase. Subsequently, individual plants grown from the obtained seeds are self-pollinated to produce seeds of a segregating population. The segregating population may include individuals that have the Mi-1 gene and the Cf-5 gene linked in the coupling phase after chromosome recombination between the two genes. Such individuals are obtained through selection using, for example, DNA markers or inoculation tests.

The individuals with the Mi-1 gene and the Cf-5 gene linked in the coupling phase can be selected from the progeny obtained by crossing with a selection method using the sequences of a genomic region including the two genes as DNA markers. Such markers include markers amplified with primers having the base sequences of SEQ ID NOs: 3 to 9. More specifically, the presence of the Mi-1 gene can be determined based on the genotyping method with the primer set of SEQ ID NOs: 3 and 4 and/or the primer set of SEQ ID NOs: 5, 6, and 7. The presence of the Cf-5 gene can also be determined based on the genotyping method with the primer set of SEQ ID NOs: 8 and 9. Such confirmation can also be performed by, for example, partially reading the genomic sequence to confirm that the genome includes the nucleotide sequences encoding the polypeptides of the amino acid sequences of SEQ ID NOs: 1 and 2.

More specifically, the above method can identify an individual homozygous for the Mi-1 gene and heterozygous for the Cf-5 gene or an individual heterozygous for the Mi-1 gene and homozygous for the Cf-5 gene as having a DNA fragment comprising the Mi-1 gene and the Cf-5 gene linked in the coupling phase in one chromosome of the homologous chromosomes. The above method can also identify an individual homozygous for both the Mi-1 gene and the Cf-5 gene as having the DNA fragment comprising the Mi-1 gene and the Cf-5 gene linked in the coupling phase on both of the homologous chromosomes.

These confirmation methods can also be used in back-crossing and self-pollination for fixing the two genes, and the progeny resistant to root-knot nematode and leaf mold can be selected.

The tomato plant resistant to root-knot nematode and leaf mold according to one or more embodiments of the present disclosure may be the progeny of F1 individuals obtained by crossing a tomato plant having the Mi-1 gene and the Cf-5 gene in the coupling phase and a tomato plant other than the tomato plant having the Mi-1 gene and the Cf-5 gene in the coupling phase. For example, a tomato plant having the Mi-1 gene and the Cf-5 gene in the coupling phase and a tomato plant other than the tomato plant having the Mi-1 gene and the Cf-5 gene in the coupling phase are crossed to produce F1 seeds. Individual plants grown from the F1 seeds are back-crossed with the individual tomato plant other than the tomato plants having the Mi-1 gene and the Cf-5 gene in the coupling phase, or are self-pollinated to produce seeds. Individuals each having the Mi-1 gene and the Cf-5 gene in the coupling phase are then selected from individual plants grown from the produced seeds. The selected individual plants are self-pollinated or crossed with tomato plants, and individuals each having the Mi-1 gene and the Cf-5 gene in the coupling phase are selected from the progeny produced by the crossing or the self-pollination. By repeating the breeding and selection, the root-knot nematode resistance and the leaf mold resistance can be introduced into any tomato plant. Self-pollination, crossing with tomato plants, cultivation, and seed production may follow a common method for cultivating tomatoes.

Individuals each having the Mi-1 gene and the Cf-5 gene in the coupling phase are selected from the progeny plants by, for example, examining the root-knot nematode resistance and the leaf mold resistance. More specifically, the root-knot nematode resistance is examined by inoculating the root-knot nematode species described above to the roots of tested individual plants and examining root gall generation. The tested individual plants with mild root galls are evaluated as resistant to the inoculated root-knot nematode species. The leaf mold resistance is tested by inoculating Passalora fulva onto the leaves of tested individual plant and examining leaf mold symptom on the leaves. A known race group of Passalora fulva that cannot infect tomato plants expressing the Cf-5 gene includes races 2, 4, 2.4, 9, 2.9, 4.9, and 2.4.9. The leaf mold resistance can be examined using at least one of these races. For example, leaf mold race 2.4.9 infects tomato plants expressing the resistance genes Cf-2, Cf-4, and Cf-9, and does not infect tomato plants expressing the Cf-5 gene. Leaf mold race 2.4.9 is thus one of the races appropriate for confirming the resistance based on the Cf-5 gene.

In a preferred aspect of the present disclosure, a breeding method according to one or more embodiments of the present disclosure includes determining the genotype of the Mi-1 gene based on the genotyping method with the primer set of SEQ ID NOs: 3 and 4 and/or the primer set of SEQ ID NOs: 5, 6, and 7, and determining the genotype of the Cf-5 gene based on the genotyping method with the primer set of SEQ ID NOs: 8 and 9, and can further include confirming that the genome comprises the nucleotide sequences encoding the polypeptides of the amino acid sequences of SEQ ID NOs: 1 and 2 by, for example, partially reading the genomic sequence.

The DNA marker herein includes a DNA sequence on a chromosome that serves as an indicator for distinguishing chromosome fragments including alleles of different sequences from one another. Examples of the DNA marker include a single nucleotide polymorphism (SNP) marker, a simple sequence repeat (SSR) marker, a restriction fragment length polymorphism (RFLP) marker, a sequence characterized amplified region (SCAR) marker, a cleaved amplified polymorphic sequence (CAPS) marker, and a KASP^{™} marker.

The DNA marker can be detected with a common method. For example, DNA extracted from each of the individual plants is used as a template in a nucleic acid amplification reaction using primers that can specifically hybridize with a specific SNP or SSR and a polymerase. Electrophoresis or another method is used to detect the presence or absence of amplification products and distinguish polymorphisms. Polymorphisms can also be distinguished by treating the DNA extracted from each of the individual plants with a restriction enzyme and detecting the pattern of resultant DNA fragments with electrophoresis or another method. The primer that can specifically hybridize with a specific SNP or SSR sequence is designed with, based on the base sequence of the genomic DNA of tomato or the SNP or SSR base sequence to be detected, a common method using, for example, a general-purpose primer design tool, and can be synthesized with a method well known in the art.

Specific examples of the tomato plant according to one or more embodiments of the present disclosure include plants of the accession numbers FERM BP-22476 (SSC-TOM-23-001) and FERM BP-22477 (SSC-TOM-23-002) described above. Seeds of these plants are deposited. The tomato plant according to one or more embodiments of the present disclosure has aspects based on the specific deposited plants as described below.

In other words, one or more aspects of the present disclosure also include a tomato plant comprising "a gene in which the root-knot nematode resistance Mi-1 gene and the leaf mold resistance Cf-5 gene are linked in the coupling phase" or "a gene having the root-knot nematode resistance Mi-1 gene and the leaf mold resistance Cf-5 gene linked in the coupling phase" derived from the deposited plants.

Comprising herein may be rephrased as having. In other words, the terms comprising and having herein are used equivalently.

One or more aspects of the present disclosure also include a tomato plant comprising "a gene in which the root-knot nematode resistance Mi-1 gene and the leaf mold resistance Cf-5 gene are linked in the coupling phase" or "a gene having the root-knot nematode resistance Mi-1 gene and the leaf mold resistance Cf-5 gene linked in the coupling phase" identified or represented by the deposited plants.

The gene, genetic trait, base sequence, chromosome fragment, or DNA fragment identified or represented by the deposited plants herein includes a gene that functions as, although not being perfectly identical to, a gene, a genetic trait, a base sequence, a chromosome fragment, or a DNA fragment in the deposited plants.

One or more aspects of the present disclosure also include a tomato plant comprising "a gene in which the root-knot nematode resistance Mi-1 gene and the leaf mold resistance Cf-5 gene are linked in the coupling phase" or "a gene having the root-knot nematode resistance Mi-1 gene and the leaf mold resistance Cf-5 gene linked in the coupling phase" that can be found in the deposited plants.

The gene, genetic trait, base sequence, chromosome fragment, or DNA fragment that "can be found" in the deposited plants herein includes a gene that has the same function as, although not being perfectly identical to, a gene, a genetic trait, a base sequence, a chromosome fragment, or a DNA fragment that "is found" in the deposited plants.

Aspects of the present disclosure also include a hybrid plant obtained from, as a parental line, a tomato plant that is identified by the accession number FERM BP-22476 or FERM BP-22477 and has the root-knot nematode resistance Mi-1 gene and the leaf mold resistance Cf-5 gene in the coupling phase, or a tomato plant that is the progeny of the hybrid plants and has the root-knot nematode resistance Mi-1 gene and the leaf mold resistance Cf-5 gene in the coupling phase.

The tomato plant comprising the gene in which the root-knot nematode resistance Mi-1 gene and the leaf mold resistance Cf-5 gene are linked in the coupling phase or the gene having the root-knot nematode resistance Mi-1 gene and the leaf mold resistance Cf-5 gene linked in the coupling phase is derived, found in, identified, or represented by the tomato plant identified by the accession number FERM BP-22476 or FERM BP-22477. The tomato plant includes a plant comprising the genomic DNA of chromosome 6 that comprises
(1) a nucleotide sequence of a portion comprising the Mi-1 gene and the Cf-5 gene in the genomic DNA of chromosome 6 of the tomato plant identified by the accession number FERM BP-22476 or FERM BP-22477, or
(2) a nucleotide sequence that is 50% or greater identical to the nucleotide sequence defined in (1) and comprises the nucleotide sequences encoding the polypeptides of the amino acid sequences of SEQ ID NOs: 1 and 2.

The nucleotide sequence defined in (1) or (2) can be included to express the polypeptides of the amino acid sequences of SEQ ID NOs: 1 and 2.

In (1), the portion comprising the Mi-1 gene and the Cf-5 gene in the genomic DNA of chromosome 6 of the tomato plant identified by the accession number FERM BP-22476 or FERM BP-22477 is preferably a portion of the genomic DNA included in the range from a base identified by ch06_2151188 to a base identified by ch06_2742590, or more preferably a portion of the genomic DNA from the base identified by ch06_2151188 to the base identified by ch06_2742590.

The nucleotide sequence defined in (2) is more preferably 60% or greater, more preferably 70% or greater, more preferably 80% or greater, more preferably 90% or greater, more preferably 95% or greater, more preferably 97% or greater, or most preferably 99% or greater identical to the nucleotide sequence defined in (1). However, the identity is less than 100%. In (2), the nucleotide sequence encoding the polypeptide of the amino acid sequence of SEQ ID NO: 1 includes an exon portion that is preferably 70% or greater, more preferably 80% or greater, more preferably 90% or greater, more preferably 95% or greater, more preferably 97% or greater, or most preferably 100% identical to a partial nucleotide sequence from the 87th base to the 3860th base of the DNA sequence that is an mRNA sequence of SEQ ID NO: 10 with all U replaced by T. Further in (2), the nucleotide sequence encoding a polypeptide of the amino acid sequence of SEQ ID NO: 2 includes an exon that is preferably 70% or greater, more preferably 80% or greater, more preferably 90% or greater, more preferably 95% or greater, more preferably 97% or greater, or most preferably 100% identical to a partial nucleotide sequence from the 654th base to the 3560th base in SEQ ID NO: 11.

Another preferred aspect of the present disclosure relates to a part of a plant body of a tomato plant having the Mi-1 gene and the Cf-5 gene in the coupling phase. The tomato plant having the Mi-1 gene and the Cf-5 gene in the coupling phase preferably has aspects described above. A part of a plant body is defined as described above. In the present aspect, a preferred example of the part of a plant body of the tomato plant having the Mi-1 gene and the Cf-5 gene in the coupling phase includes a tomato plant cell that comprises a DNA fragment comprising the nucleotide sequences encoding the polypeptides of the amino acid sequences of SEQ ID NOs: 1 and 2 and has no regeneration potential. Examples of the DNA fragment include the above fragment as a DNA fragment comprising the Mi-1 gene and the Cf-5 gene linked in the coupling phase. Specific aspects of the nucleotide sequences encoding the polypeptides of the amino acid sequences of SEQ ID NOs: 1 and 2 are as described above. In the tomato plant cell in the present aspect, the DNA fragment may be located in any portion and may be in or outside the chromosome. However, the DNA fragment is preferably located as a part of chromosome DNA in the chromosome. In the tomato plant cell in the present aspect, examples of the tomato plant cell without regeneration potential include cells other than seeds in a tomato fruit and cells in the epicarp (including epidermal cells), the mesocarp, endocarp, placenta, locules, vascular bundle, pedicle, or sepals of the tomato fruit. The tomato plant cell in the present aspect is preferably isolated from the plant body.

A healthy product herein is a highly pure tomato product, which is described in a production method (described later), including a plant body, a seed, a seedling, or a portion of the plant body of a tomato plant that has the Mi-1 gene and the Cf-5 gene in the coupling phase and is at least free from infection of the root-knot nematode to which the Mi-1 gene is resistant and the leaf mold race to which the Cf-5 gene is resistant.

Tomato F1 products are commonly prepared, often by artificial hand-pollination. Although a pollination operator conducts operations such as identification of flowers appropriate for F1 production, emasculation, pollination, and isolation while washing and disinfecting the hands and tools as appropriate to avoid contamination of unintended pollen based on protocols and also the operator's experience, F1 products may have lower purity due to mistakes in crossing or unexpected accidents in logistics.

However, customers including seedling producers, farmers, consumers, and other purchasers of tomato products always expect F1 products with 100% purity. Commonly, seed companies fulfill such responsibility in processing and selling products by conducting direct or indirect purity tests before selling the products. Tomatoes are to be grown for about four months before their fruits are harvestable and also have a long harvestable period of about five months. Thus, fresh produce damaged due to not having 100% purity directly harms the interests of the purchasers greatly.

Purity tests must be accurate, rapid, and inexpensive because of their direct connection to the production cost. Thus, a product seller is to supply healthy products that can greatly reduce the number of testing steps, save work hours and workforce, and have 100% purity to fulfill the expectations of the customers.

The healthy products include a form of seeds, seedlings (sprouts), entire plant bodies, and parts of plant bodies, and further include various forms including packaged products of these healthy products wrapped appropriately with wrapping materials such as containers, bags, and trays.

In other words, one aspect of the healthy product in one or more embodiments of the present disclosure can be, for example, selling the tomato plant having the Mi-1 gene and the Cf-5 gene in the coupling phase or its portion wrapped with a wrapping material such as a container, a bag, or a tray after confirming that the plant or the portion is free from infection of the root-knot nematode to which the Mi-1 gene is resistant and the leaf mold race to which the Cf-5 gene is resistant.

### <DNA Fragment comprising Mi-1 Gene and Cf-5 Gene>

Another embodiment of the present disclosure relates to a DNA fragment comprising the Mi-1 gene and the Cf-5 gene.

The DNA fragment according to the present embodiment incorporated into a nucleic acid vector as appropriate can be introduced into a tomato plant. The DNA fragment according to the present embodiment includes the Mi-1 gene and the Cf-5 gene in a manner in which each gene can be expressed in the cells of the tomato plant.

The Mi-1 gene can comprise the nucleotide sequence encoding the polypeptide of the amino acid sequence of SEQ ID NO: 1. The Cf-5 gene can comprise the nucleotide sequence encoding the polypeptide of the amino acid sequence of SEQ ID NO: 2.

The DNA fragment according to the present embodiment particularly preferably comprises
(1) a nucleotide sequence of a portion comprising the Mi-1 gene and the Cf-5 gene in the genomic DNA of chromosome 6 in a tomato plant identified by the accession number FERM BP-22476 or FERM BP-22477, or
(2) a nucleotide sequence that is 50% or greater identical to the nucleotide sequence defined in (1) and comprises the nucleotide sequences encoding the polypeptides of the amino acid sequences of SEQ ID NOs: 1 and 2.

Specific aspects of the nucleotide sequences defined in (1) and (2) are as described above.

The DNA fragment according to the present embodiment is preferably isolated. The DNA fragment according to the present embodiment is also preferably artificially developed.

### <Method for Producing Tomato Plant Resistant to Root-Knot Nematode and Leaf Mold>

The method for breeding a tomato plant that is resistant to root-knot nematode and leaf mold and has the Mi-1 gene and the Cf-5 gene in the coupling phase comprises, as described above, crossing the tomato plant having the Mi-1 gene and the Cf-5 gene in the coupling phase as a parental material to introduce the root-knot nematode resistance and the leaf mold resistance into an intended tomato plant. Thus, a new resistant tomato plant is produced.

Once a plant with the root-knot nematode resistance Mi-1 gene and the leaf mold resistance Cf-5 gene linked in the coupling phase is bred, chromosome recombination between the two genes is highly unlikely to happen. After breeding the plant described above, progeny plants of the bred plant are assessed, with common DNA assays or inoculation assays, for a single trait alone that is selected from the group consisting of the root-knot nematode resistance, the leaf mold resistance, the presence of the Mi-1 gene, and the presence of the Cf-5 gene. This can reliably assess whether each progeny plant has the traits of both the root-knot nematode resistance and the leaf mold resistance.

The method according to one or more embodiments of the present disclosure comprises assessing solely a single trait in the four traits consisting of the root-knot nematode resistance, the leaf mold resistance, the presence of the Mi-1 gene, and the presence of the Cf-5 gene for confirming whether both the Mi-1 gene and the Cf-5 gene are present. This can greatly reduce the number of assessment processes and save work hours and workforce. An example of such an advantage is described below. When inoculation selections with root-knot nematode and leaf mold on the same individuals are conducted in a breeding process, each individual selected by root-knot nematode inoculation is then inoculated with leaf mold as described later. The leaf mold inoculation is performed after a portion including a growing point is cut out from the tomato plant inoculated with root-knot nematode and incubated for about one month based on conditions. Typical tomato breeding processes further involve selection of other traits as well to breed tomato plants with traits that are more acceptable than known traits. When a plant having the root-knot nematode resistance gene Mi-1 and the leaf mold resistance gene Cf-5 in the coupling phase is bred, progeny plants obtained from the plant as a parental line can skip examination of one of the root-knot nematode resistance or the leaf mold resistance. Each individual can thus avoid the time lag of about one month as described above. This can accelerate breeding.

The two genes linked in the coupling phase are inherited to the progeny in a manner similar to a single gene, and thus easily introduced into an intended line. This can further accelerate tomato variety breeding. Further, the tomato plant having the root-knot nematode resistance gene Mi-1 and the leaf mold resistance gene Cf-5 in the coupling phase according to one or more embodiments of the present disclosure used as a crossing parent allows F1 varieties to easily accumulate useful genes such as the powdery mildew resistance gene or the Tomato yellow leaf curl virus resistance gene located on chromosome 6.

In one or more embodiments of the present disclosure, cross breeding using a tomato plant having the Mi-1 gene and the Cf-5 gene in the coupling phase as a parental line can be used in the method for producing a tomato plant having resistance to root-knot nematode and leaf mold and having an Mi-1 gene and a Cf-5 gene in the coupling phase. One specific example of the method comprises a first crossing process of crossing a tomato plant having the Mi-1 gene and the Cf-5 gene in the coupling phase with an unspecified first tomato plant; a second crossing process of obtaining a segregating population by self-pollination or back-crossing an F1 individual obtained by the first crossing process or by intraspecifically or interspecifically crossing the F1 individual with a second tomato plant different from the first tomato plant used as a parental line in the first crossing; and a selection process of selecting a tomato plant having the Mi-1 gene and the Cf-5 gene in the coupling phase from the segregating population. A common tomato cultivation method can be used for self-pollination, back-crossing, interspecific crossing, intraspecific crossing, cultivating tomatoes, and producing seeds.

The tomato plant having the Mi-1 gene and the Cf-5 gene in the coupling phase used as a parental line in the first crossing process can be the tomato plant resistant to root-knot nematode and leaf mold according to one or more embodiments of the present disclosure described above. In one or more embodiments of the present disclosure, the tomato plant having the Mi-1 gene and the Cf-5 gene in the coupling phase used as a parental line is preferably a tomato plant that is resistant to root-knot nematode and leaf mold and has the Mi-1 gene and the Cf-5 gene linked in the coupling phase along with the traits as a cultivated variety. Such a tomato plant resistant to root-knot nematode and leaf mold is represented by line SSC-TOM-23-001, individuals obtained from line SSC-TOM-23-001 used as a parental line, line SSC-TOM-23-002, individuals obtained from line SSC-TOM-23-002 used as a parental line, and their progeny tomato plants with root-knot nematode resistance and leaf mold resistance derived from the Mi-1 gene and the Cf-5 gene.

The first tomato plant used as a parental line in the first crossing process or the second tomato plant used as a parental line for interspecific or intraspecific crossing in the second crossing process is more preferably, but not limited to, a cultivated tomato plant having at least one disease resistance gene other than the Mi-1 gene and the Cf-5 gene. Still more preferably, the tomato plant having at least one disease resistance gene that is on chromosome 6 and is other than the Mi-1 gene and the Cf-5 gene may be used as a parental line to allow relatively easier construction of a new tomato line that has, in addition to the Mi-1 gene and the Cf-5 gene on chromosome 6, additional disease resistance genes.

In the selection process, individuals each having the Mi-1 gene and the Cf-5 gene in the coupling phase are selected using the DNA markers that are located near the respective Mi-1 and Cf-5 genes in chromosome 6 of tomato plants. The DNA markers or specific methods to be used can be the same as the construction of the individuals each having the Mi-1 gene and the Cf-5 gene in the coupling phase using the DNA markers described above. When the first tomato plant used as a parental line in the first crossing process or the second tomato plant used as a parental line for interspecific or intraspecific crossing in the second crossing process has neither Mi-1 gene nor Cf-5 gene, individuals in the segregating population that are homozygous or heterozygous for the two genes can be selected as the individuals each having the Mi-1 gene and the Cf-5 gene in the coupling phase. When the first tomato plant used as a parental line in the first crossing process or the second tomato plant used as a parental line for interspecific or intraspecific crossing in the second crossing process has the Mi-1 gene or the Cf-5 gene, the segregating population includes an individual that is heterozygous for the Mi-1 gene and homozygous for the Cf-5 gene, an individual that is homozygous for the Mi-1 gene and heterozygous for the Cf-5 gene, and an individual that is homozygous for both the Mi-1 gene and the Cf-5 gene. These individuals can thus be selected as the individuals each having the Mi-1 gene and the Cf-5 gene in the coupling phase.

To select individual tomato plants that have additional disease resistance in addition to root-knot nematode resistance and leaf mold resistance, selection based on DNA markers or inoculation assays that can determine whether another disease resistance gene is present can be performed in addition to the selection of individuals each having the Mi-1 gene and the Cf-5 gene in the coupling phase as described above. The DNA markers that can determine whether another disease resistance gene is present can be, for example, SNP located in and near the gene loci of the other disease resistance genes.

Subsequently, seeds produced from the selected progeny individuals are repeatedly crossed to obtain a tomato plant resistant to root-knot nematode and leaf mold that has more stable traits and can be cultivated as a crop stable in, for example, germination rate, yield, or seed productivity. Crossing may be repeated any number of times, more than once, two to three times, or more than three times.

### <Method for Producing Healthy Products Comprising Tomato Plants According to Embodiments of the Present Disclosure>

One or more embodiments of the present disclosure provides a method for producing a highly pure healthy product that comprises a plant body, a seed, a seedling, or a portion of the plant body of a tomato plant. The method can comprise confirming one trait selected from the group consisting of the root-knot nematode resistance, the leaf mold resistance, the presence of the Mi-1 gene, and the presence of the Cf-5 gene in the tomato plant according to one or more embodiments of the present disclosure, and preparing a product comprising a plant body, a seed, a seedling, or a portion of the plant body of the tomato plant confirmed to have the trait described above.

As described above, the tomato plant according to one or more embodiments of the present disclosure includes the root-knot nematode resistance Mi-1 gene and the leaf mold resistance Cf-5 gene in the coupling phase. In a preferred aspect, once a plant with the root-knot nematode resistance Mi-1 gene and the leaf mold resistance Cf-5 gene linked in the coupling phase is obtained, a product using the plant and its progeny plant is assessed solely for a single trait selected from the group consisting of the root-knot nematode resistance, the leaf mold resistance, the presence of the Mi-1 gene, and the presence of the Cf-5 gene with a DNA assay or an inoculation assay based on a common method, and can also be reliably assessed for the presence or absence of the other traits.

This method can assess purity by solely confirming a single trait selected from the group consisting of the root-knot nematode resistance, the leaf mold resistance, the presence of the Mi-1 gene, and the presence of the Cf-5 gene. This can greatly reduce the number of ordinary assessment processes and can save work hours and workforce. This method can thus provide a product including a tomato plant with higher commercial value. Customers, such as seedling producers or farmers, who purchase the product including the tomato plant according to one or more embodiments of the present disclosure can eliminate damage to vegetables and fruits due to, for example, unintended contamination or crossing in advance.

A sample used in a process of confirming the trait described above can be a seed or a sprout of a tomato plant, or a portion of the seed or the sprout. For example, a seed of a tomato plant is prepared and partially taken as a sample for the process described above. When the trait is confirmed in the process, the remaining portion of the seed of the tomato plant can be used in a product as a seed with indirect confirmation of the trait. When a seedling of a tomato plant is prepared, its sprout or a portion of the sprout can be taken as a sample for the process. When the trait is confirmed in the process, the seedling of the tomato plant can be used in a product as a seedling with direct or indirect confirmation of the trait.

In this method, a product comprising a plant body, a seed, a seedling, or a portion of the plant body of a tomato plant is typically a plant body, a seed, a seedling, or a portion of the plant body of a tomato plant with confirmation of the above trait and a wrapping material, such as a container, a bag, or tray, that contains the plant body, the seed, the seedling, or the portion of the plant body of the tomato plant. For example, the product is a packaged product comprising a seed of a tomato plant and a wrapping material containing the seed and a product comprising a seedling of a tomato plant and a tray containing the seedling. In this method, preparing a plant body, a seed, a seedling, or a portion of the plant body of a tomato plant with confirmation of the above trait includes, for example, wrapping the plant body, the seed, the seedling, or the portion of the plant body of the tomato plant with confirmation of the trait with a wrapping material into a packaged product. The product may be refrigerated as appropriate or proceed to further processing.

### Examples

### [Example 1]

### <Inoculation Test for Root-Knot Nematode Resistance Examination>

The root-knot nematode resistance of a tomato plant is determined with a method described below with reference to Non-Patent Literature 14.

Southern root-knot nematodes (Meloidogyne incognita) were collected in Japan, and a strain from a single egg mass was propagated in soil. Susceptible tomato plants were cultivated for two months in the soil. Then, second-stage juveniles were collected from root galls formed on tomato roots and the soil. A suspension of 100 nematodes/ml was prepared with sterile water and used for inoculation.

Tomato seeds were sowed on a commercial gardening soil. Fourteen days after sowing, the young tomato seedlings were transplanted to 9-cm pots containing sterile soil. The plants were cultivated for 14 days in a greenhouse controlled at 22 to 28 °C, to prepare the tomato plants for bioassay tests.

In each test, a tomato plant was irrigated with 50 ml of the nematode suspension on the soil near the plant base. The plants were further cultivated for 35 days. After cultivation, the root of each tomato plant was washed and examined for disease development.

Disease development was rated based on a five-scale root gall index for tomatoes described in Non-Patent Literature 14. Ratings of 0 and 1 are determined as resistant, and ratings of 2 to 4 are determined as susceptible.
Rating 0: No root galls.
Rating 1: Root galls are slightly observed but damage is not noticeable.
Rating 2: Root galls are observed. Few large or connected root galls.
Rating 3: Many large and small root galls. Thickened roots covered with root galls are less than or equal to 50% of the total root area.
Rating 4: Many roots are covered with root galls and thickened.

### <Inoculation Test for Leaf Mold Resistance Examination>

The leaf mold resistance of a tomato plant was determined with the method described below with reference to Non-Patent Literature 15.

Leaf mold race 2.4.9 collected in Japan was incubated on a potato dextrose agar (PDA) plate at 23 °C for two weeks. After the incubation, conidia were collected from plate colonies. A conidial suspension of 1 × 10⁵ conidia/mL was prepared with sterile water and used for inoculation.

Tomato seeds were sowed on a commercial gardening soil. 14 days after sowing, the young tomato seedlings were transplanted to 9-cm pots containing sterile soil. The plants were cultivated for 14 days in a greenhouse controlled at 22 to 28 °C, to prepare the tomato plants for bioassay tests.

For inoculation of root-knot nematode and leaf mold on a single tomato plant, a shoot was cut out from a tomato plant inoculated with root-knot nematodes. The cut shoot was placed into sterile soil and grown for 28 days in a greenhouse set to 22 to 28 °C to develop roots. The cuttings produced were used for inoculation of leaf mold.

In each test, a tomato plant was sprayed with 3 mL of the conidial suspension on the back of its leaves and was cultivated for 18 days in a growth chamber set at 23 °C and 80 to 100% humidity. After the cultivation, symptoms on the leaves of the test tomato plants were observed and examined for disease development.

Disease development was checked based on the race differentiation for leaf mold described in Non-Patent Literatures 5 and 8. The determination was made as susceptible or resistant.
Susceptible: Many mycelium spots
Resistant: No mycelium spots

### [Example 2]

To develop a tomato plant with the Mi-1 gene and the Cf-5 gene linked in the coupling phase, the inventors first prepared SKTom-A and SKTom-C, which are large-fruit tomato lines homozygous for the Mi-1 gene and susceptible to leaf mold race 2.4.9, and SKTom-B and SKTom-D, which are large-fruit tomato lines homozygous for the Cf-5 gene and susceptible to root-knot nematodes (all lines are owned by Sakata Seed Corporation). The crossing performed consisted of a combination of SKTom-A and SKTom-B and a combination of SKTom-C and SKTom-D, and seeds were obtained from large-fruit tomato F1 plants that were heterozygous for the two genes in repulsion phase. These F1 tomato plants were grown and self-pollinated to produce F2 seeds. Table 5 shows characteristics of the parental lines SKTom-A, SKTom-B, SKTom-C, and SKTom-D.

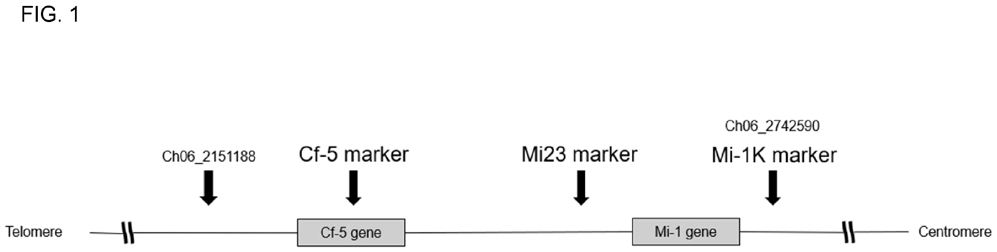

To develop an individual with the Mi-1 gene and the Cf-5 gene linked in the coupling phase from a tomato plant population obtained by sowing the F2 seeds, the inventors started genotyping assays using DNA markers. The DNA markers used for determining genotypes for the Mi-1 gene and the Cf-5 gene are shown below (Table 1). FIG. 1 is a schematic diagram illustrating the positional relationship between the Mi-1 gene, the Cf-5 gene, and the respective DNA markers. In Non-Patent Literature 6, the Cf-5 gene is identified as being at a more centromeric position than the Cf-2.2 gene. The most centromeric base of the Cf-2.2 gene is a base identified by Ch06_2151188 on the reference genome based on Sol Genomics Network. The Cf-5 gene is thus located at a more centromeric position than the most centromeric base of the Cf-2.2 gene (FIG. 1).

**[Table 1]**

| Marker | Primer | Sequence | Restriction enzyme | Marker type | SEQ ID NO |
|---|---|---|---|---|---|
| Mi23 | Mi23_F | TGGAAAAATGTTGAATTTCT TTTG | - | co-dominant SCAR | 3 |
| | Mi23_R | GCATACTATATGGCTTGTTTA CCC | | | 4 |
| Mi-1K | Mi1K_1F | GAAGGTGACCAAGTTCATG CTGAAGAGAATTAGATTGC ACTAACCC | - | co-dominant KASP^{™} | 5 |
| | Mi1K_2F | GAAGGTCGGAGTCAACGGA TTGAAGAGAATTAGATTGC ACTAACCA | | | 6 |
| | Mi1K_R | TAAGGCATCAAAGGCGTTA GA | | | 7 |
| Cf-5 | Cf5_F | AGCAGATGAAATCCCTCGG TC | TaqI | co-dominant CAPS | 8 |
| | Cf5_R | CCTCGCTGCTTCTTTCTCCT T | | | 9 |

Of the markers shown in Table 1, Mi23 is a marker designed near the Mi-1 gene as described in the above Non-Patent Literature 2. Genotyping is thus performed based on a method described in Non-Patent Literature 2.

Of the markers shown in Table 1, Mi-1K herein is located opposite (centromeric) to Mi23 with the Mi-1 gene between them. Mi-1K is a KASP^{™} marker that detects SNP at Ch06_2742590. Mi1K_1F and Mi1K_2F each have a sequence with its 3'-end being one base that hybridizes with the SNP target to be distinguished. The Mi-1K marker is designed based on the complementary strand sequence of the sequence indicated by the reference genome SL4.0. Thus, on the sequence indicated by the reference genome SL4.0, one base at the 3'-end of Mi1K_1F distinguishes an SNP sequence with G (guanine), and one base at the 3'-end of Mi1K_2F distinguishes an SNP sequence with T (thymine). Similarly to Mi23, Mi-1K is designed to allow specific detection of the Mi-1 gene in various lines.

When the genomic DNA of an individual plant that does not possess the Mi-1 gene is used as a template, PCR using Mi1K_1F as the forward primer and Mi1K_R as the reverse primer yields amplified products. When the genomic DNA of an individual plant that does not possess the Mi-1 gene is used as a template, PCR using Mi1K_2F as the forward primer and Mi1K_R as the reverse primer yields amplified products. SNP genotypes are distinguished based on whether amplified products are produced. In this manner, a KASP^{™} assay using the three types of oligo-DNA as one primer set allows distinguishing SNP genotypes.

For example, a tomato plant homozygous for the Mi-1 gene has the SNP genotype homozygous for the allele with G (guanine) at the position identified with ch06_2742590. A susceptible line does not possess the Mi-1 gene and has the SNP genotype homozygous for the allele with T (thymine) at the position identified with ch06_2742590. An F1 that is heterozygous for the Mi-1 gene has the SNP genotype of G/T at the position identified with ch06_2742590, and heterozygously has the Mi-1 type allele with G for the SNP and a susceptible allele with T for the SNP.

As described above, both the Mi23 marker and the Mi-1K marker, are located near the Mi-1 gene with the Mi-1 gene between them. Individuals each having the Mi-1 gene are thus reliably obtained by selecting, from the segregating population, individuals each showing the Mi-1 genotype for both of the two markers.

Of the markers shown in Table 1, a Cf-5 marker is a marker designed based on a specific sequence of the Cf-5 gene in Non-Patent Literature 7 described above. Individuals each having the Cf-5 gene are thus reliably obtained by selecting individuals each showing the Cf-5 genotype through genotyping based on the method described in Non-Patent Literature 7.

The Mi23 marker and the Cf-5 marker were used for DNA assays of an F2 population derived from the multiple lines described above. The results showed that, among a total of 1899 individuals, two individuals each had a marker genotype having the Mi-1 gene and the Cf-5 gene in the coupling phase. In subsequent assays using the Mi-1K marker, the two individuals also had the root-knot nematode resistance genotype for the Mi-1K marker. Further, the two individuals were, respectively, a combination of the breeding lines SKTom-A and SKTom-B and a combination of breeding lines SKTom-C and SKTom-D. These breeding lines are, as shown in Table 5, different from one another in genetic backgrounds and characteristics. Thus, the results suggested that the Mi-1 gene and the Cf-5 gene can be linked in the coupling phase independently of genetic backgrounds and characteristics. Subsequently, self-pollinated progeny of each individual was used to develop individuals that are homozygous for the two genes, which were named lines SKTom-E and SKTom-F. SKTom-E is appropriate for the winter season and exhibits drooping leaves. In contrast, SKTom-F is appropriate for the summer and autumn seasons and has a compact plant form with upright and small leaves. These two lines were inoculated with the southern root-knot nematode and leaf mold race 2.4.9, and their resistance to the two diseases was confirmed. The results are shown in Table 2.

**[Table 2]**

| **Line** | **Marker genotype^{*1}** | | **Phenotype for southern root-knot nematode inoculation** | | | | | | **Phenotype for leaf mold race 2.4.9 inoculation** | | |
|---|---|---|---|---|---|---|---|---|---|---|---|
| | | | **Resistant** | | **Susceptible** | | | **Total number of individuals** | **Resistant** | **Susceptible** | **Total number of individuals** |
| | **Mi23** | **Cf-5** | **Rating 0** | **Rating 1** | **Rating 2** | **Rating 3** | **Rating 4** | | **No mycelium spots** | **Many mycelium spots** | |
| SKTom-A | A/A | B/B | 10 | 0 | 0 | 0 | 0 | 10 | 0 | 5 | 5 |
| SKTom-B | B/B | A/A | 0 | 0 | 0 | 0 | 10 | 10 | 5 | 0 | 5 |
| SKTom-C | A/A | B/B | 6 | 2 | 0 | 0 | 0 | 8 | 0 | 8 | 8 |
| SKTom-D | B/B | A/A | 0 | 0 | 0 | 0 | 8 | 8 | 8 | 0 | 8 |
| SKTom-E | A/A | A/A | 11 | 2 | 0 | 0 | 0 | 13 | 13 | 0 | 13 |
| SKTom-F*² | A/A | A/A | 11 | 13 | 0 | 0 | 0 | 24 | 24 | 0 | 24 |
| Manalbo | | | | | | | | | 0 | 8 | 8 |
| (Cf-0) | | | | | | | | | | | |
| Vetomold (Cf-2) | | | | | | | | | 0 | 8 | 8 |
| Purdue135 (Cf-4) | | | | | | | | | 0 | 8 | 8 |
| IVT1149 (Cf-5) | | | | | | | | | 8 | 0 | 8 |
| Ontario7818 (Cf-6) | | | | | | | | | 8 | 0 | 8 |
| IVT1154 (Cf-9) | | | | | | | | | 0 | 8 | 8 |

| | | | | | | | | | | | |
|---|---|---|---|---|---|---|---|---|---|---|---|
| *1 A/A: Homozygous for the resistance allele, B/B: Homozygous for the Susceptible allele, A/B: Heterozygous for the resistance and susceptible alleles *2 Accession number FERM BP-22477 | | | | | | | | | | | |

### [Example 3]

Subsequently, the line SKTom-E was crossed with the line SKTom-G susceptible to root-knot nematode and leaf mold (owned by Sakata Seed Corporation) to produce F1 tomato plants. The F1 plants were then self-pollinated to produce seeds for an F2 population. The seeds were sowed and the F2 tomato population was grown. Assays using the DNA markers and inoculation were performed on the plants of the F2 population, and revealed that the Mi-1 gene and the Cf-5 gene were inherited while being linked in the coupling phase. The results are shown in Table 3.

**[Table 3]**

| **Line** | **Marker genotype^{*1}** | | **Phenotype for southern root-knot nematode inoculation** | | | | | | **Phenotype for leaf mold race 2.4.9 inoculation** | | |
|---|---|---|---|---|---|---|---|---|---|---|---|
| | | | **Resistant** | | **Susceptible** | | | **Total number of individuals** | **Resistant** | **Susceptible** | **Total number of individuals** |
| | **Mi23** | **Cf-5** | **Rating 0** | **Rating 1** | **Rating 2** | **Rating 3** | **Rating 4** | | **No myceliu m spots** | **Many mycelium spots** | |
| SKTom-G | B/B | B/B | 0 | 0 | 0 | 4 | 4 | 8 | 0 | 8 | 8 |
| SKTom-G x SKTom-E (F1) | A/B | A/B | 4 | 4 | 0 | 0 | 0 | 8 | 8 | 0 | 8 |
| SKTom-G x SKTom-E (F2) | A/A | A/A | 27 | 4 | 0 | 0 | 0 | 31 | 31 | 0 | 31 |
| | A/B | A/B | 38 | 24 | 0 | 0 | 0 | 62 | 62 | 0 | 62 |
| | B/B | B/B | 0 | 0 | 1 | 8 | 18 | 27 | 0 | 27 | 27 |
| SKTom-H^{*2} | A/A | A/A | 15 | 9 | 0 | 0 | 0 | 24 | 24 | 0 | 24 |

| | | | | | | | | | | | |
|---|---|---|---|---|---|---|---|---|---|---|---|
| *1 A/A: Homozygous for the resistance allele, B/B: Homozygous for the susceptible allele, A/B: Heterozygous for the resistance and susceptible alleles *2 Accession number FERM BP-22476 | | | | | | | | | | | |

Individuals homozygous for both the Mi-1 gene and the Cf-5 gene were selected using the DNA markers from tomato plants obtained by sowing the seeds for an F2 population. Similarly to Example 2, the individuals were further inoculated with the southern root-knot nematode and leaf mold race 2.4.9 to confirm resistance to the two diseases and were named line SKTom-H (Table 3).

Line SKTom-F (accession number FERM BP-22477, identification labeling: SSC-TOM-23-002) and line SKTom-H (accession number FERM BP-22476, identification labeling: SSC-TOM-23-001) with the Mi-1 gene and the Cf-5 gene linked in the coupling phase were obtained through the tests described above and their seeds were deposited.

### [Example 4]

In Examples 2 and 3, the tomato plants that were resistant to root-knot nematode and leaf mold and possessed the Mi-1 gene and the Cf-5 gene linked in the coupling phase were successfully developed from the two lines with genetically different backgrounds and traits. These lines were both large-fruit tomato lines. Thus, development of tomato plants resistant to root-knot nematode and leaf mold was also performed with cherry tomato lines.

A cherry tomato line SKTom-I that is homozygous for the Mi-1 gene and does not possess the Cf-5 gene was crossed with a cherry tomato line SKTom-J that is homozygous for the Cf-5 gene and does not possess the Mi-1 gene to produce an F2 cherry tomato population. The lines are both owned by Sakata Seed Corporation. Similarly, a cherry tomato line SKTom-K that is homozygous for the Mi-1 gene and does not possess the Cf-5 gene was crossed with a cherry tomato line SKTom-L that is homozygous for the Cf-5 gene and does not possess the Mi-1 gene to produce an F2 cherry tomato population. The lines are both owned by Sakata Seed Corporation. Table 5 shows characteristics of the respective parental lines.

DNA assays using the Mi23 marker and the Cf-5 marker were performed for 4262 individual plants of the F2 population obtained by crossing the lines SKTom-I and SKTom-J. The results revealed that two of the 4262 individuals were heterozygous for the Mi-1 gene and homozygous for the Cf-5 gene. One of the 4262 individuals was homozygous for the Mi-1 gene and heterozygous for the Cf-5 gene. In other words, this suggested that the Mi-1 gene and the Cf-5 gene were successfully linked in the coupling phase on one of the homologous chromosomes of chromosome 6 in each of these three individuals. These three individuals were then each tested using the Mi-1K marker and showed that its genotype of the Mi-1 gene was the same as that of the Mi23 marker.

DNA assays using the Mi23 marker and the Cf-5 marker were then performed on 1719 individual plants in the F2 population obtained by crossing the lines SKTom-K and SKTom-L. The results revealed that one of the 1719 individuals was heterozygous for the Mi-1 gene and homozygous for the Cf-5 gene. Another one of the 1719 individuals was homozygous for the Mi-1 gene and heterozygous for the Cf-5 gene. In other words, this suggested that the Mi-1 gene and the Cf-5 gene were successfully linked in the coupling phase on one of the homologous chromosomes of chromosome 6 in each of these two individuals. These two individuals were then each tested using the Mi-1K marker and showed that its genotype of the Mi-1 gene was the same as that of the Mi23 marker.

In the two cherry tomato F2 populations described above, a total of five individuals had marker genotypes showing that they had the Mi-1 gene and the Cf-5 gene in the coupling phase. Subsequently, individuals homozygous for the two genes were selected from self-pollinated progeny of each of the five individuals, obtaining lines SKTom-M, SKTom-N, SKTom-O, SKTom-P, and SKTom-Q.

Similarly to Examples 2 and 3, these five lines were inoculated with the southern root-knot nematode and leaf mold race 2.4.9 to confirm resistance to the two diseases. The results are shown in Table 4.

**[Table 4]**

| **Line** | **Marker genotype^{*1}** | | **Phenotype for southern root-knot nematode inoculation** | | | | | | **Phenotype for leaf mold race 2.4.9 inoculation** | | |
|---|---|---|---|---|---|---|---|---|---|---|---|
| | | | **Resistant** | | **Susceptible** | | | **Total number of individuals** | **Resistant** | **Susceptible** | **Total number** of **individuals** |
| | **Mi23** | **Cf-5** | **Rating 0** | **Rating 1** | **Rating 2** | **Rating 3** | **Rating 4** | | **No mycelium spots** | **Many mycelium spots** | |
| SKTom-I | A/A | B/B | 5 | 3 | 0 | 0 | 0 | 8 | 0 | 8 | 8 |
| SKTom-J | B/B | A/A | 0 | 0 | 0 | 8 | 0 | 8 | 8 | 0 | 8 |
| SKTom-K | A/A | B/B | 7 | 1 | 0 | 0 | 0 | 8 | 0 | 8 | 8 |
| SKTom-L | B/B | A/A | 0 | 0 | 0 | 1 | 7 | 8 | 8 | 0 | 8 |
| SKTom-M | A/A | A/A | 5 | 4 | 0 | 0 | 0 | 9 | 9 | 0 | 9 |
| SKTom-N | A/A | A/A | 1 | 3 | 0 | 0 | 0 | 4 | 4 | 0 | 4 |
| SKTom-O | A/A | A/A | 7 | 2 | 0 | 0 | 0 | 9 | 9 | 0 | 9 |
| SKTom-P | A/A | A/A | 3 | 3 | 0 | 0 | 0 | 6 | 6 | 0 | 6 |
| SKTom-Q | A/A | A/A | 4 | 7 | 0 | 0 | 0 | 11 | 11 | 0 | 11 |

| | | | | | | | | | | | |
|---|---|---|---|---|---|---|---|---|---|---|---|
| *1 A/A: Homozygous for the resistance allele, B/B: Homozygous for the susceptible allele, A/B: Heterozygous for the resistance and susceptible alleles | | | | | | | | | | | |

The results described above revealed that the root-knot nematode resistance gene Mi-1 and the leaf mold resistance gene Cf-5 can be linked in the coupling phase in various tomato plants with genetically different backgrounds, as well as in large-fruit tomato lines. Similarly, the results revealed that the Mi-1 gene and the Cf-5 gene once linked in the coupling phase are inherited in a manner similar to a single gene in various tomato plants with genetically different backgrounds, as well as in large-fruit tomato lines.

**[Table 5]**

| **Line** | **Marker genotype^{*1}** | | **Type** | **Fruit color** | **Characteristics** |
|---|---|---|---|---|---|
| | **Mi23** | **Cf-5** | | | |
| SKTom-A | A/A | B/B | Large | Pink | Fruit 180-200 g, Non-self topping, Upright leaves, Moderate number of flowers, Short internode, Moderate leaf color, Adaptable for summer and autumn cropping |
| SKTom-B | B/B | A/A | Large | Pink | Fruit 220-240 g, Non-self topping, Horizontal to nodding leaves, Fewer flowers, Long internode, Moderate leaf color, Adaptable for winter survival |
| SKTom-C | A/A | B/B | Large | Pink | Fruit 200-220 g, Non-self topping, Horizontal to nodding leaves, Fewer flowers, Short internode, Slightly light leaf color, Adaptable for summer and autumn cropping |
| SKTom-D | B/B | A/A | Large | Pink | Fruit 180-200 g, Non-self topping, Upright leaves, Moderate number of flowers, Moderate length of internode, Dark green leaf color, Adaptable for summer and autumn cropping |
| SKTom-G | B/B | B/B | Large | Red | Fruit 200-220 g, Self topping, Horizontal to nodding leaves, Moderate number of flowers, Short internode, Moderate leaf color |
| SKTom-I | A/A | B/B | Cherry-sized | Red | Fruit 12-14 g, Non-self topping, Horizontal leaves, Relatively fewer flowers, Very short internode, Dark green leaf color, Glossy fruits |
| SKTom-J | B/B | A/A | Cherry-sized | Red | Fruit 10-12 g, Non-self topping, Horizontal leaves, Relatively many flowers, Short internode, Dark green leaf color |
| SKTom-K | A/A | B/B | Cherry-sized | Red | Fruit 10-12 g, Non-self topping, Horizontal leaves, Moderate number of flowers, Short internode, Dark green leaf color |
| SKTom-L | B/B | A/A | Cherry-sized | Red | Fruit 12-14 g, Non-self topping, Horizontal leaves, Moderate number of flowers, Moderate length of internode, Dark green leaf color, Glossy fruits |

| | | | | | |
|---|---|---|---|---|---|
| *1 A/A: Homozygous for the resistance allele, B/B: Homozygous for the susceptible allele | | | | | |

### [Example 5]

Subsequently, seeds of the deposited line (accession number FERM BP-22476) developed in Example 3 were germinated on a 72-cell tray, and tomato plant seedlings were prepared. As a control, seeds of SKTom-C, which is the line homozygous for Mi-1 and not possessing Cf-5, were germinated in the same manner on a 72-cell tray, and tomato plant seedlings were prepared.

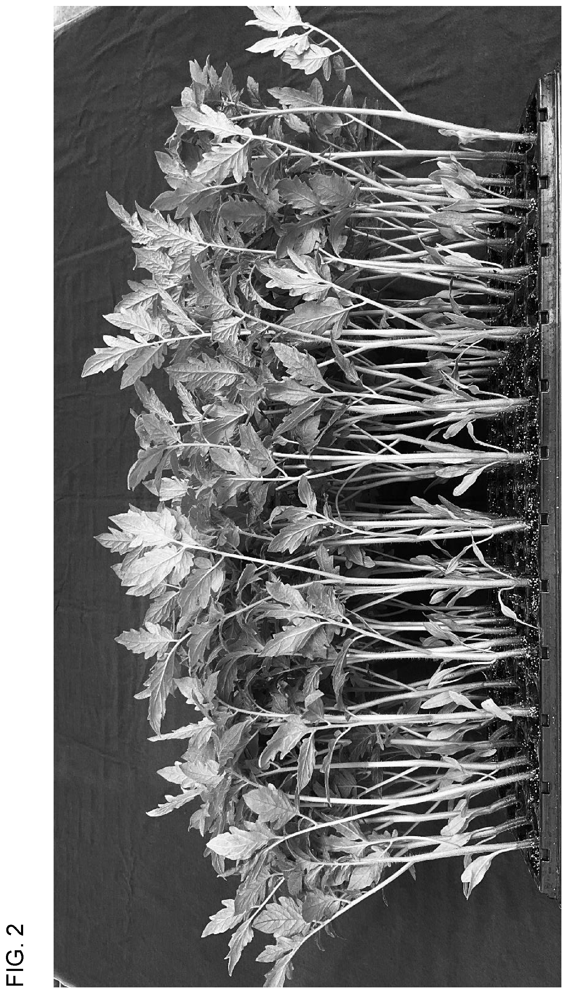

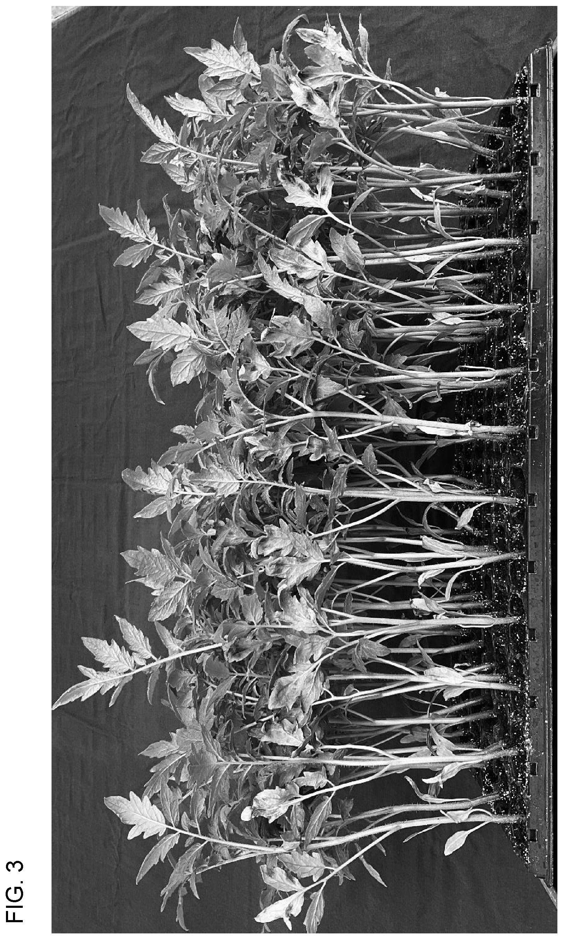

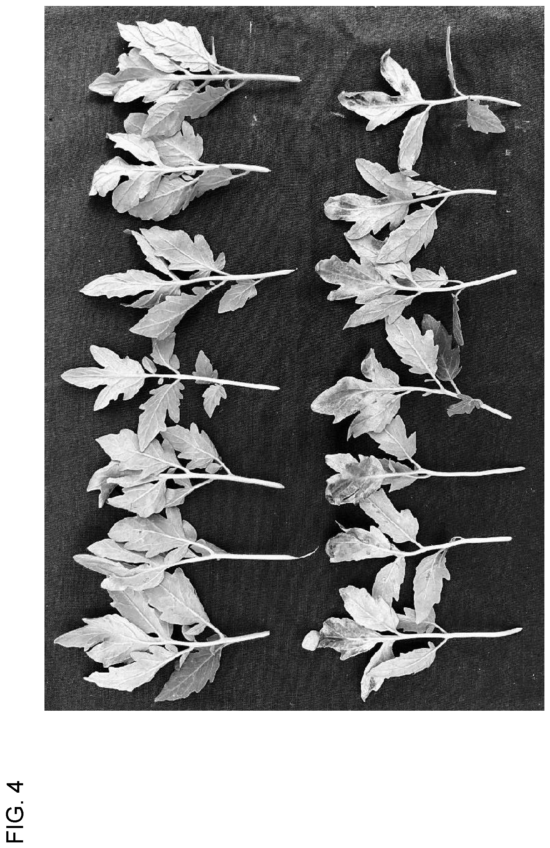

The prepared tomato plant seedlings were inoculated with leaf mold race 2.4.9 and examined for disease development 18 days later. The seedlings of deposited line (accession number FERM BP-22476) grown in the trays showed no symptoms and maintained the value of tomato plant products (FIGs. 2 and 4). In contrast, the seedlings of SKTom-C grown in the trays showed severe symptoms, and lost their value as tomato plant products due to the leaf mold infection (FIGs. 3 and 4).

The prepared tomato plants were tested for the Mi-1 gene using the Mi23 marker, and all individuals of the two lines were homozygous for the Mi-1 gene. This revealed that, as is apparent in Examples 3, 4, and 5, the Cf-5 gene can be detected by confirming the genotype for the Mi-1 gene alone in the lines, or typically the deposited lines, with the Mi-1 gene and the Cf-5 gene linked in the coupling phase.

## Claims

1. A tomato plant having resistance to root-knot nematode and leaf mold, the tomato plant comprising:
an Mi-1 gene conferring resistance to root-knot nematode; and
a Cf-5 gene conferring resistance to leaf mold,
the Mi-1 gene and the Cf-5 gene being in the coupling phase.

2. The tomato plant according to claim 1, wherein
the tomato plant comprises a DNA fragment comprising
a nucleotide sequence encoding a polypeptide of an amino acid sequence of SEQ ID NO: 1, and
a nucleotide sequence encoding a polypeptide of an amino acid sequence of SEQ ID NO: 2.

3. The plant according to claim 1 or claim 2, wherein
the Mi-1 gene and the Cf-5 gene are located on chromosome 6.

4. The tomato plant according to any one of claims 1 to 3, wherein
the tomato plant comprises a nucleotide sequence encoding a polypeptide of an amino acid sequence of SEQ ID NO: 1 and a nucleotide sequence encoding a polypeptide of an amino acid sequence of SEQ ID NO: 2 on a same genomic DNA of chromosome 6.

5. The plant according to any one of claims 1 to 4, wherein
the tomato plant is homozygous or heterozygous for the Mi-1 gene, and
the tomato plant is homozygous or heterozygous for the Cf-5 gene.

6. The plant according to any one of claims 1 to 5, wherein
the Mi-1 gene is identified with a genotyping method using a primer set comprising a primer comprising a nucleotide sequence of SEQ ID NO: 3 and a primer comprising a nucleotide sequence of SEQ ID NO: 4 and/or a genotyping method using a primer set comprising a primer comprising a nucleotide sequence of SEQ ID NO: 5, a primer comprising a nucleotide sequence of SEQ ID NO: 6, and a primer comprising a nucleotide sequence of SEQ ID NO: 7, and
the Cf-5 gene is identified with a genotyping method using a primer set comprising a primer comprising a nucleotide sequence of SEQ ID NO: 8 and a primer comprising a nucleotide sequence of SEQ ID NO: 9.

7. The plant according to any one of claims 1 to 6, wherein
the tomato plant is resistant to at least one species of root-knot nematodes in a genus Meloidogyne and resistant to at least one race in a Passalora fulva race group that cannot infect tomato plants expressing the Cf-5 gene.

8. The tomato plant according to any one of claims 1 to 7, wherein
the tomato plant has root-knot nematode resistance and leaf mold resistance derived from a tomato plant identified by an accession number FERM BP-22476 or FERM BP-22477.

9. The tomato plant according to any one of claims 1 to 8, wherein
the tomato plant is a hybrid obtained from a tomato plant identified by an accession number FERM BP-22476 or FERM BP-22477 as a parental line, or progeny of the hybrid plant.

10. The tomato plant according to any one of claims 1 to 9, wherein
the tomato plant comprises genomic DNA of chromosome 6 comprising
(1) a nucleotide sequence of a portion comprising the Mi-1 gene and the Cf-5 gene in the genomic DNA of chromosome 6 of a tomato plant identified by an accession number FERM BP-22476 or FERM BP-22477, or
(2) a nucleotide sequence being 50% or greater identical to the nucleotide sequence defined in (1) and comprising a nucleotide sequence encoding a polypeptide of an amino acid sequence of SEQ ID NO: 1 and a nucleotide sequence encoding a polypeptide of an amino acid sequence of SEQ ID NO: 2.

11. The tomato plant according to claim 10, wherein
the portion comprising the Mi-1 gene and the Cf-5 gene in the genomic DNA of chromosome 6 of the tomato plant identified by the accession number FERM BP-22476 or FERM BP-22477 is included in a region from a base identified by ch06_2151188 to a base identified by ch06_2742590 in the genomic DNA of chromosome 6 of the tomato plant identified by the accession number FERM BP-22476 or FERM BP-22477.

12. A portion of a plant body of the tomato plant according to any one of claims 1 to 11.

13. The portion according to claim 12, wherein
the portion is a tomato plant cell without regeneration potential, and the tomato plant cell comprises a DNA fragment comprising a nucleotide sequence encoding a polypeptide of an amino acid sequence of SEQ ID NO: 1 and a nucleotide sequence encoding a polypeptide of an amino acid sequence of SEQ ID NO: 2.

14. A fruit of the tomato plant according to any one of claims 1 to 11.

15. A seed of the tomato plant according to any one of claims 1 to 11.

16. A DNA fragment, comprising:
an Mi-1 gene; and
a Cf-5 gene.

17. The DNA fragment according to claim 16, wherein
the DNA fragment comprises a nucleotide sequence encoding a polypeptide of an amino acid sequence of SEQ ID NO: 1 and a nucleotide sequence encoding a polypeptide of an amino acid sequence of SEQ ID NO: 2.

18. The DNA fragment according to claim 16 or claim 17, wherein
the DNA fragment comprises
(1) a nucleotide sequence of a portion comprising the Mi-1 gene and the Cf-5 gene in the genomic DNA of chromosome 6 of the tomato plant identified by the accession number FERM BP-22476 or FERM BP-22477, or
(2) a nucleotide sequence being 50% or greater identical to the nucleotide sequence defined in (1) and comprising a nucleotide sequence encoding a polypeptide of an amino acid sequence of SEQ ID NO: 1 and a nucleotide sequence encoding a polypeptide of an amino acid sequence of SEQ ID NO: 2.

19. The DNA fragment according to claim 18, wherein
the portion comprising the Mi-1 gene and the Cf-5 gene in the genomic DNA of chromosome 6 of the tomato plant identified by the accession number FERM BP-22476 or FERM BP-22477 is included in a region from a base identified by ch06_2151188 to a base identified by ch06_2742590 in the genomic DNA of chromosome 6 of the tomato plant identified by the accession number FERM BP-22476 or FERM BP-22477.

20. A method for producing F1 seeds from the tomato plant according to any one of claims 1 to 11, the method comprising:
crossing the tomato plant according to any one of claims 1 to 11 with another of the tomato plant according to any one of claims 1 to 11 or with a plant capable of being crossed with the tomato plant according to any one of claims 1 to 11; and
harvesting F1 seeds from an individual obtained by the crossing.

21. A method for producing a tomato plant having resistance to root-knot nematode and leaf mold and comprising an Mi-1 gene and a Cf-5 gene in the coupling phase, the method comprising:
self-pollinating the tomato plant according to any one of claims 1 to 11; and
further repeatedly self-pollinating an individual obtained by the self-pollinating for one or more generations.

22. A method for producing a tomato plant having resistance to root-knot nematode and leaf mold and comprising an Mi-1 gene and a Cf-5 gene in the coupling phase, the method comprising:
crossing the tomato plant according to any one of claims 1 to 11 with a first tomato plant;
obtaining a segregating population by self-pollinating or back-crossing an F1 individual obtained by the crossing or by interspecifically or intraspecifically crossing the F1 individual with a second tomato plant different from the first tomato plant used in the crossing; and
selecting a tomato plant having the Mi-1 gene and the Cf-5 gene in the coupling phase from the segregating population.

23. The method according to claim 22, wherein
the selecting comprises at least one of
detecting the Mi-1 gene with a genotyping method using a primer set comprising a primer comprising a nucleotide sequence of SEQ ID NO: 3 and a primer comprising a nucleotide sequence of SEQ ID NO: 4,
detecting the Mi-1 gene with a genotyping method using a primer set comprising a primer comprising a nucleotide sequence of SEQ ID NO: 5, a primer comprising a nucleotide sequence of SEQ ID NO: 6, and a primer comprising a nucleotide sequence of SEQ ID NO: 7,
detecting the Cf-5 gene with a genotyping method using a primer set comprising a primer comprising a nucleotide sequence of SEQ ID NO: 8 and a primer comprising a nucleotide sequence of SEQ ID NO: 9,
confirming resistance to root-knot nematode, or
confirming resistance to leaf mold.

24. A method for manufacturing a product comprising a plant body, a seed, a seedling, or a portion of the plant body of a tomato plant, the method comprising:
confirming that the tomato plant according to any one of claims 1 to 11 has one trait selected from the group consisting of root-knot nematode resistance, leaf mold resistance, presence of an Mi-1 gene, and presence of a Cf-5 gene; and
preparing a product comprising a plant body, a seed, a seedling, or a portion of the plant body of the tomato plant confirmed to have the one trait.

25. The method according to claim 24, wherein
the product comprises a seed of the tomato plant and a wrapping material containing the seed, or the product comprises a seedling of the tomato plant and a tray containing the seedling.

26. A product comprising a plant body, a seed, a seedling, or a portion of the plant body of a tomato plant manufactured with the method according to claim 24.
